(19) Europäisches Patentamt
European Patent Office
Office européen des brevets

(11) Publication number: **0 040 408**
**B1**

(12) **EUROPEAN PATENT SPECIFICATION**

(45) Date of publication of patent specification: **29.08.84**

(51) Int. Cl.$^3$: **C 07 D 487/04** // A61K31/40 ,(C07D487/04, 205/00, 209/00)

(21) Application number: **81103719.1**

(22) Date of filing: **14.05.81**

(54) **Beta-lactam compounds and process for production thereof.**

(30) Priority: **16.05.80 JP 64032/80**

(43) Date of publication of application:
**25.11.81 Bulletin 81/47**

(45) Publication of the grant of the patent:
**29.08.84 Bulletin 84/35**

(84) Designated Contracting States:
**DE FR GB IT**

(56) References cited:
**EP-A-0 001 628**
**EP-A-0 008 514**
**EP-A-0 008 888**
**EP-A-0 011 173**

(73) Proprietor: **SANRAKU-OCEAN CO., LTD.**
**15-1, Kyobashi 1-chome Chuo-ku**
**Tokyo (JP)**

(72) Inventor: **Yoshioka, Takeo**
**Ayase Green Heights 3-3102, 1959**
**Kamitsuchidana, Kanagawa-ken, Ayase (JP)**
Inventor: **Yamamoto, Kenichi**
**Shonan Life Town G-2-2, 1120, Endo**
**Kanagawa-ken, Fujisawa (JP)**
Inventor: **Kato, Yasuyuki**
**Take-So, 1960, Arima, Takatsu-ku**
**Kanagawa-ken, Kawasaki (JP)**
Inventor: **Shimauchi, Yasutaka**
**1-101-13, Yurigaoka, Ninomiya-machi**
**Kanagawa-ken, Naka-gun (JP)**
Inventor: **Ishikura, Tomoyuki**
**640, Kowada**
**Kanagawa-ken, Chigasaki (JP)**

(74) Representative: **Kraus, Walter, Dr. et al**
**Patentanwälte Kraus & Weisert Irmgardstrasse 15**
**D-8000 München 71 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 040 408 B1**

**Description**

This invention relates to a novel process for producing 7-oxo-1-azabicyclo[3.2.0.]hept-2-ene-2-carboxylic acid derivatives, and more specifically, to a versatile process for producing specific $\beta$-lactam compounds and salts thereof.

Compounds of the following formula

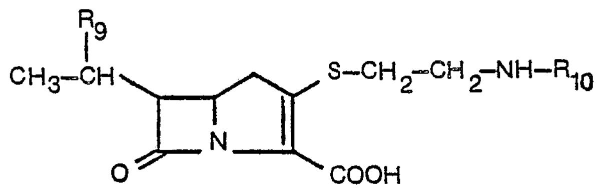

(VI)

wherein $R_9$ represents a hydrogen atom or a methyl or hydroxyl group, and $R_{10}$ represents a hydrogen atom or an acetyl group,

have recently been developed as antibiotics produced by fermentative methods utilizing microorganisms such as *Streptomyces cattleya* (NRRL 8057) or *Streptomyces* sp. A271 (ATCC 31358, FERM—P 3984). Investigations have been actively undertaken in an attempt to obtain derivatives of these compounds which have excellent antimicrobial activity and/or excellent stability. In particular, compounds of the above formula (VI) in which $R_9$ represents a hydrogen atom or a methyl group were developed by the present inventors. As a result of various investigations relating to a method for converting the S-side chain at the 3-position of the compounds of formula (VI) to other groups, they have found that by changing the S-side chain of the 3-position of the compounds of formula (VI) to a SH group and reacting the resulting product with an organic halide, there can be easily obtained compounds corresponding to formula (VI) which have a side chain corresponding to the organic halide used.

EP—A—11 173 describes novel antibiotic 7-oxo-1-azabicylo[32.20]hept-2-ene-2-carboxylic acid derivatives. Thienamycin derivatives having antibiotic activity are described in EP—A—1628 and EP—A—8888.

Thus, according to this invention, there is provided a process for producing a compound of the general formula

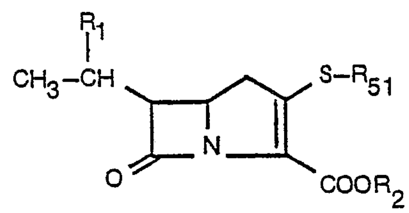

(I—1)

wherein $R_1$ represents a hydrogen atom, a methyl group, or a hydroxyl group, $R_2$ represents a hydrogen atom or an ester residue selected from

(1) a $C_1$—$C_{14}$ alkyl or $C_2$—$C_6$ alkenyl group which may be substituted by a halogen atom, a hydroxyl group, a $C_1$—$C_6$ alkoxy group, a $C_1$—$C_6$ alkylthio group, a $C_3$—$C_{12}$ cycloalkyloxy group, a $C_3$—$C_{13}$ cycloalkylthio group, a phenoxy group (the phenyl group may further be substituted by a halogen atom, a $C_1$—$C_6$ alkoxy group, a $C_1$—$C_6$ haloalkyl group or a nitro group), a phenylthio group (the phenyl group may further be substituted by a halogen atom, a $C_1$—$C_6$ alkoxy group, a $C_1$—$C_6$ haloalkyl group or a nitro group), a $C_2$—$C_{16}$ acyl group, a $C_2$—$C_{14}$ alkoxycarbonyl group (the alkoxy group may further be substituted by a halogen atom), and a $C_8$—$C_{24}$ aralkyloxycarbonyl groups (the aralkyl group may further be substituted by a halogen atom, a $C_1$—$C_6$ alkoxy group, a $C_1$—$C_6$ haloalkyl group, or a nitro group);

(2) $C_3$—$C_{15}$ cycloalkyl group;

(3) a phenyl, tolyl, xylyl or naphthyl group which may be substituted by a halogen atom, a $C_1$—$C_6$ alkoxy group, a $C_1$—$C_6$ haloalkyl group, a $C_1$—$C_6$ alkanoylamino group, a $C_1$—$C_6$ alkoxycarbonyl group or a nitro group; and

(4) a $C_1$—$C_6$ alkyl group substituted by 1—3 phenyl groups, and phenyl groups may further be substituted by 1—3 substituents selected from a halogen atom, a $C_1$—$C_6$ alkyl group, a $C_1$—$C_6$ alkoxy group, a $C_1$—$C_6$ haloalkyl group, a $C_1$—$C_6$ alkoxycarbonyl group, a hydroxyl group and a nitro group,

and $R_{51}$ represents a monovalent organic group selected from groups (1) to (4) as defined for $R_2$, or its salt, which comprises reacting a compound of the general formula

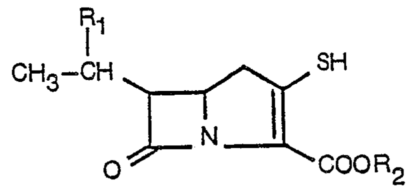

(II)

wherein $R_1$ and $R_2$ are as defined above, or its salt, with a diazo compound of the formula

$$R_4 = N_2 \qquad \text{(III-2)}$$

wherein $R_4$ represents a divalent group remaining after removal of one hydrogen atom present on the carbon atom at the 1-position from a corresponding $R_{51}$ monovalent organic group in which the carbon atom at the 1-position is a primary or secondary carbon atom.
The invention also provides a process for producing a compound of the general formula

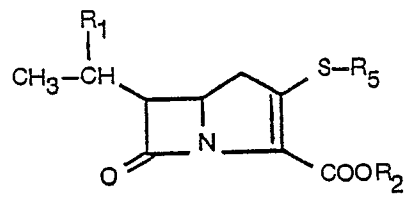

(I)

wherein $R_1$ represents a methyl group, $R_2$ is as defined above and $R_5$ represents the group $R_3$ or —CO—$R_3$ in which $R_3$ represents a monovalent organic group selected from groups (1) to (4) as defined for $R_2$, or its salt, which comprises reacting a compound of the general formula

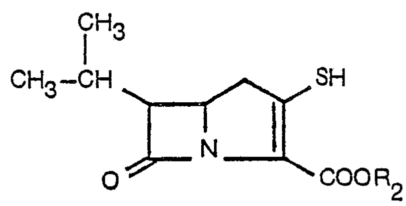

(II—1)

wherein $R_2$ is as defined above, or its salt, with a compound of the formula

$$R_3 — X \qquad \text{(III-1)}$$

wherein $R_3$ is as defined above, and X represents a halogen atom or an activated carboxyl group.
This invention also provides new compounds of the formula

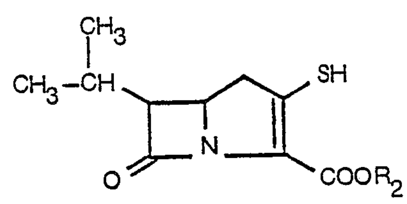

(I—a)

wherein $R_2$ is as defined above and its salt.

These compounds are novel compounds which are useful as intermediates for producing the antibacterially active compounds of the formulae (I) and (I1).

It should be noted in this connection that the reaction of the compound of formula (II) (in which $R_1$ represents groups defined above other than methyl) with the compound of formula (III-1) is the subject matter of EP—A—0,024,832 and is excluded from the scope of the claims of the present application.

Typical examples of $C_1$—$C_{14}$ alkyl groups substituted by the above mentioned groups include the following.

2,2,2-Trichloroethyl,
2,2,2-trifluoroethyl,
hydroxyethyl,

2-hydroxypropyl,
methyoxymethyl,
ethoxymethyl,
isopropoxymethyl,
ethoxyethyl,
ethoxypropyl,
methylthiomethyl,
ethylthiomethyl,
ethylthioethyl,
methylthiopropyl,
isopropylthioethyl,
cyclohexyloxymethyl,
cyclohexylthiomethyl,
phenoxymethyl,
phenoxyethyl,
p-chlorophenoxymethyl,
p-methoxyphenoxymethyl,
p-trifluoromethylphenoxymethyl,
p-nitrophenoxymethyl,
phenylthiomethyl,
p-methoxyphenylthiomethyl,
p-trifluoromethylphenylthiomethyl,
p-nitrophenylthiomethyl,

acetoxymethyl,
acetoxyethyl,
propionyloxymethyl,
acetoxypropyl,
pivaloyloxymethyl,

acetylthioethyl,
acetonyl,
propionylmethyl,
phenacyl,
p-chlorophenacyl,
p-bromophenacyl,
p-methylphenacyl,
p-tert-butylphenacyl,
p-ethylphenacyl,
p-nitrophenacyl,
o-nitrophenacyl,
p-trifluoromethylphenacyl,
p-methoxyphenacyl,
benzoylethyl,
phenylacetonyl,
methoxycarbonylmethyl,

carbamoylethyl,
tert-butoxycarbonylmethyl,
tert-amyloxycarbonylmethyl,
2,2,2-trichloroethoxycarbonylmethyl,
2,2,2-tribromoethoxycarbonylmethyl,
benzyloxycarbonylmethyl,
diphenylmethoxycarbonylmethyl,
triphenylmethoxycarbonylmethyl,
p-nitrobenzyloxycarbonylmethyl,
p-nitrobenzyloxycarbonylethyl,
p-methoxybenzyloxycarbonylmethyl, and
phenethyloxycarbonylmethyl.

Suitable cycloalkyl groups have 3 to 15 carbon atoms, preferably 3 to 10 carbon atoms, more preferably 3 to 6 carbon atoms. Such cycloalkyl groups include those having a lower alkyl groups on the ring. Typical examples of such cycloalkyl groups are cyclopropyl, cyclobutyl, cyclopentyl, cyclohexyl, cycloheptyl, cyclooctyl, 4-methylcyclohexyl, and 4-ethylcyclohexyl.

Specific examples of phenyl, tolyl, xylyl, and naphthyl groups which are substituted by the above mentioned groups are p-chlorophenyl, p-methoxyphenyl, p-acetoxyphenyl, p-acetylaminophenyl, p-methoxycarbonylphenyl, and p-nitrophenyl.

In general formula (III-1), suitable halogen atoms for X are chlorine, bromine and iodine atoms. The term "activated carboxyl group" denotes a residue left after removal of the monovalent organic group $R_3$ from an ester-forming active derivative of an organic carboxylic acid ($R_3COOH$) capable of forming a thioester bond

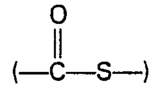

by reaction with the thiol group (SH) of the thiol compound. Examples of the ester-forming active derivatives of the organic carboxylic acid ($R_3COOH$) are given below.

(a) Acid halides, imidazolides or pyrazolides of the formula $R_3COX_1$ wherein $X_1$ represents a halogen atom, imidazole or methylpyrazole.

(b) Acid anhydrides of the formula

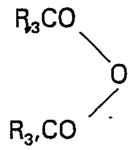

wherein $R_3$ and $R_{3'}$ are identical or different and represent a monovalent organic group, and when they are different, the acid anhydrides are mixed acid anhydrides.

(c) Active esters of the formula $R_3COOA$ wherein A represents

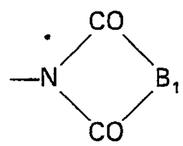

(wherein $B_1$ represents a lower alkylene group, a lower alkenylene group, or a phenyl group) or —$COOB_2$ (wherein $B_2$ represents a lower alkyl group, or a substituted or unsubstituted benzyl group).

When $R_2$ in the compound formulae (I) and (II) is a hydrogen atom, the compounds of formulae (I) and (II) may be in the form of salts. Examples of such salts include inorganic salts such as sodium, potassium, magnesium, calcium and ammonium salts, and salts with organic bases such as monoethylamine, dimethylamine, triethylamine, monoethanolamine, diethanolamine, benzathine and procaine.

Specific examples of the compounds of formula (II) used as starting materials in the process of this invention, other than those given in Examples hereinbelow, are as follows:

Methyl 6-ethyl-3-mercapto-7-oxo-1-azabicyclo[3.2.0]hept-2-ene-2-carboxylate,
benzhydryl 6-ethyl-3-mercapto-7-oxo-1-azabicyclo[3.2.0]hept-2-ene-2-carboxylate,
methyl 6-isopropyl-3-mercapto-7-oxo-1-azabicyclo[3.2.0] -2-ene-2-carboxylate,
benzyl 6-isopropyl-3-mercapto-7-oxo-1-azabicyclo[3.2.0]hept-2-ene-2-carboxylate,
benzhydryl 6-isopropyl-3-mercapto-7-oxo-1-azabicyclo[3.2.0]hept-2-ene-2-carboxylate,
pivaloyloxymethyl 6-isopropyl-3-mercapto-7-oxo-1-azabicyclo[3.2.0]hept-2-ene-2-carboxylate,
methyl 6-(1-hydroxy)ethyl-3-mercapto-7-oxo-1-azabicyclo[3.2.0]hept-2-ene-2-carboxylate,
benzyl 6-(1-hydroxy)ethyl-3-mercapto-7-oxo-1-azabicyclo[3.2.0]hept-2-ene-2-carboxylate,
benzhydryl 6-(1-hydroxy)ethyl-3-mercapto-7-oxo-1-azabicyclo[3.2.0]hept-2-ene-2-carboxylate,
and
pivaloyloxymethyl 6-(1-hydroxy)ethyl-3-mercapto-7-oxo-1-azabicyclo[3.2.0]hept-2-ene-2-carboxylate.

Examples of the compound of formula (III-1) or (III-2), which is the other starting material used in the process of this invention include the following.

[A] Organic halide compounds (the compounds of formula (III-1) in which X is halogen)
Propyl iodide,
methyl chloroacetate,
ethyl bromoacetate

chloromethoxymethane,
chloromethylthiomethane,

[B] Organic diazo compounds (the compounds of formula (III-2)

Diazomethane, diazoethane, phenyldiazomethane, diphenyldiazomethane, and diazoglycine methyl ester.

The reaction of the compound of formula (II) or its salt with the compound of formula (III-1) or (III-2) can sometimes be carried out in the absence of a solvent. Generally, however, it is carried out suitably in an inert organic solvent. Examples of useful solvents are ethers such as dioxane, tetrahydrofuran, diethyl ether and glyme, hydrocarbons such as benzene, toluene and xylene, esters such as ethyl acetate and butyl acetate, and amides such as N,N-dimethyl formamide (DMF), hexamethylphosphoramide (HMPA) and dimethyl sulfoxide (DMSO).

There is no particular restriction on the reaction temperature, and it can be varied widely according to the types of the starting materials, etc. Generally, the reaction can be carried out at a temperature of from −50°C to +50°C, preferably at room temperature or lower temperatures.

When the halide is used as the starting material of formula (III-1), the reaction is carried out preferably in the presence of a base. Usable bases include sodium hydride, potassium hydride, sodium amide, potassium amide, sodium hydroxide, potassium hydroxide, sodium ethoxide, sodium methoxide, potassium butoxide, 1,8-diazabicyclo[5,4,0]-7-undecene, tetramethylguanidine, triethylamine, tripropylamine, collidine, methylmorpholine, and pyridine. Organic bases are preferred. Advantageously, the bases is used in an amount of at least 1 equivalent, preferably 1.0 to 2.0 equivalents, per mole of the halide.

The amount of the compound (III-1) or (III-2) is not critical, and can be varied widely depending upon the types of the starting materials, the reaction conditions, etc. Generally, the amount is at least 1.0 mole, preferably 1.1 to 2.0 moles, per mole of the compound of formula (II).

The above reaction can be carried out under atmospheric pressure, reduced pressures or elevated pressures. Generally, the use of normal atmospheric pressure is sufficient. Under the conditions described above, the present reaction can be completed generally within 0.5 to 3.0 hours.

When the compound of formula (II) in which $R_2$ is a hydrogen atom or its salt is used as a starting material, it sometimes happens that the SH group at the 3-position is exchanged with S—$R_5$ and the carboxyl group at the 2-position is esterified [especially when the compound of formula (III-1) in which X is a halogen atom or the diazo compound of formula (III-2) is used as the other starting material]. This embodiment is also included within the scope of the invention, and in this case, $R_2$ in the product of formula (I) denotes $R_3$ as an exception.

In the manner described above, the compound of formula (I) can be obtained in good yields. When the product is the compound of formula (I) in which $R_2$ is an ester residue easily splittable by hydrolysis or hydrogenolysis, such as methylthiomethyl, pivaloyloxymethyl, benzyl or p-nitrobenzyl, it is isolated and purified by the methods to be described, and then saponified or hydrogenolyzed in a customary manner to split off the group $R_2$, whereby it can be converted to a compound of the following formula or its salt.

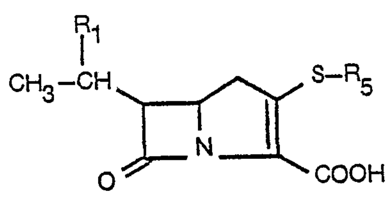

(I')

wherein $R_1$ and $R_5$ are as defined hereinabove.

Isolation and purification of the compound of formula (I) may be effected, for example, by diluting the reaction mixture with a water-immiscible inert solvent such as benzene, toluene, methylene chloride or ethyl acetate, washing the diluted reaction mixture with a phosphate buffer (pH 8.7) or an aqueous solution of sodium hydrogen carbonate and then with a phosphate buffer (pH 6.8), drying the solvent layer over anhydrous sodium sulfate, distilling the dried layer under reduced pressure, dissolving the residue in a small amount of the inert solvent, passing the solution through a column of LH-20 or Biobeads S-series (a styrene-divinylbenzene polymer, a product of Bio-Rad Company), and developing it with the same solvent, and if required, further passing the eluates through a silica gel column. Thus, the compound of formula (I) can be isolated from the reaction mixture.

The compound of formula (II) used as a starting material in the process of this invention is a novel compound not described in the prior literature. It can be produced, for example, by reacting a compound of the general formula

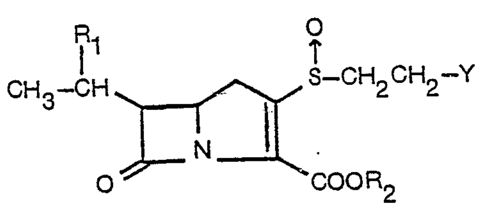

(IV)

wherein Y represents an amino group which may be protected, or its N-oxide, and $R_1$ and $R_2$ are as defined above,

or its salt, with a compound of the formula

$$MSH \qquad (V)$$

wherein M represents a hydrogen atom or an alkali metal.

The N-protecting group in the "amino group which may be protected" may be any protective groups for the amino group which have been known heretofore. Examples include the following.

(a) Substituted or unsubstituted aralkyl groups such as benzyl, p-nitrobenzyl, m-chlorobenzyl, p-methoxybenzyl, benzhydryl, and trityl.

(b) Groups of the formula $—COR_{12}$ [wherein $R_{12}$ represents a hydrogen atom, an alkyl group (e.g., a lower alkyl such as methyl, ethyl, n-propyl, isopropyl, n-butyl, and tert-butyl), a cycloalkyl group (e.g., cyclopentyl and cyclohexyl), a substituted or unsubstituted aryl group (e.g., phenyl, or substituted phenyl such as p-methoxyphenyl or p-nitrophenyl), or a substituted or unsubstituted aralkyl group (e.g., benzyl or substituted benzyl such as p-nitrobenzyl, m-chlorobenzyl, p-bromobenzyl or p-methoxybenzyl)]. Examples includes formyl, acetyl, propionyl, benzoyl, p-nitrobenzoyl, m-chlorobenzoyl, p-bromobenzoyl, phenylacetyl, p-methoxyphenylacetyl, and p-nitrophenylacetyl.

(c) Groups of the formula $—COOR_{13}$ [wherein $R_{13}$ represents an alkyl group (e.g., a lower alkyl group such as methyl, ethyl, n-propyl, isopropyl, n-butyl and tert-butyl), a cycloalkyl group (e.g., cyclopentyl and cyclohexyl), or a substituted or unsubstituted aralkyl group (e.g., benzyl or substituted benzyl such as p-nitrobenzyl, m-chlorobenzyl, p-bromobenzyl, or p-methoxybenzyl)]. Examples include methoxycarbonyl, ethoxycarbonyl, tert-butoxycarbonyl, benzyloxycarbonyl and p-nitrobenzyloxycarbonyl.

(d) Groups of the formula $—SO_2—R_{14}$ [wherein $R_{14}$ represents a lower alkyl group such as methyl, or a substituted or unsubstituted aryl group such as phenyl or tolyl]. Examples are methyl, benzenesulfonyl and tosyl.

(e) Groups of the formula

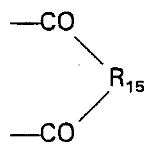

[wherein $R_{15}$ represents a lower alkylene group, a lower alkenylene group or a phenylene group], such as succinyl, maleinyl and phthalyl.

(f) Other groups such as trimethylsilyl and 4,4-dimethyl-1-oxo-2-hexen-3-yl.

Specific examples of the compound of formula (IV) other than those described in the working Examples given hereinbelow include:

p-nitrobenzyl 3-(2-phenylacetamido)ethylsulfinyl-6-ethyl-7-oxo-1-azabicyclo[3.2.0]hept-2-ene-2-carboxylate,

o-nitrobenzyl 3-(2-phenylacetamido)ethylsulfinyl-6-isopropyl-7-oxo-1-azabicyclo[3.2.0]hept-2-ene-2-carboxylate,

p-nitrobenzyl-3-(2-phenylacetamido)ethylsulfinyl-6-(1-hydroxy)ethyl-7-oxo-1-azabicyclo[3.2.0]hept-2-ene-2-carboxylate,

p-nitrobenzyl 3-(2-benzoylamino)ethylsulfinyl-6-ethyl-7-oxo-1-azabicyclo[3.2.0]hept-2-ene-2-carboxylate,

o-nitrobenzyl 3-(2-benzoylamino)ethylsulfinyl-6-isopropyl-7-oxo-1-azabicyclo[3.2.0]hept-2-ene-2-carboxylate,

p-nitrobenzyl 3-(2-benzoylamino)ethylsulfinyl-6-(1-hydroxy)ethyl-7-oxo-1-azabicyclo[3.2.0]hept-2-ene-2-carboxylate,

p-nitrobenzyl 3-(2-benzyloxycarbonylamino)ethylsulfinyl-6-ethyl-7-oxo-1-azabicyclo[3.2.0.]hept-2-ene-2-carboxylate,

o-nitrobenzyl 3-(2-benzyloxycarbonylamino)ethylsulfinyl-6-isopropyl-7-oxo-1-azabicyclo[3.2.0]hept-2-ene-2-carboxylate,

p-nitrobenzyl 3-(2-benzyloxycarbonylamino)ethylsulfinyl-6-(1-hydroxy)ethyl-7-oxo-azabicyclo[3.2.0]hept-2-ene-2-carboxylate,

p-nitrobenzyl 3-(2-tert-butyloxycarbonylamino)ethylsulfinyl-6-ethyl-7-oxo-1-azabicyclo[3.2.0]hept-2-ene-2-carboxylate,

p-nitrobenzyl 3-(2-tert-butyloxycarbonylamino)ethylsulfinyl-6-isopropyl -7-oxo-1-azabicyclo[3.2.0]hept-2-ene-carboxylate,

o-nitrobenzyl 3-(2-tert-butyloxycarbonylamino)ethylsulfinyl-6-(1-hydroxyethyl)-7-oxo-1-azabicyclo[3.2.0]hept-2-ene-2-carboxylate,

p-nitrobenzyl 3-(2-phthaloylamino)ethylsulfinyl-6-ethyl-7-oxo-1-azabicyclo[3.2.0]hept-2-ene-2-carboxylate,

p-nitrobenzyl 3-(2-phthaloylamino)ethylsulfinyl-6-(1-hydroxy)ethyl-7-oxo-1-azabicyclo[3.2.0]hept-2-ene-2-carboxylate,

sodium 3-(2-acetamido)ethylsulfinyl-6-ethyl-7-oxo-1-azabicyclo-[3.2.0]hept-2-ene-2-carboxylate,

lithium 3-(2-acetamido)ethylsulfinyl-6-isopropyl-7-oxo-1-azabicyclo[3.2.0]hept-2-ene-2-carboxylate,

sodium 3-(2-acetamido)ethylsulfinyl-6-(1-hydroxy)ethyl-7-oxo-1-azabicyclo[3.2.0]hept-2-ene-2-carboxylate,

p-nitrobenzyl 3-(2-dimethylamino)ethylsulfinyl-6-ethyl-7-oxo-1-azabicyclo[3.2.0]hept-2-ene-2-carboxylate,

o-nitrobenzyl 3-(2-dimethylamino)ethylsulfinyl-6-isopropyl-7-oxo-1-azabicyclo[3.2.0]hept-2-ene-2-carboxylate,

p-nitrobenzyl 3-(2-dimethylamino)ethylsulfinyl-6-(1-hydroxy)-ethyl-7-oxo-1-azabicyclo[3.2.0]hept-2-ene-2-carboxylate,

p-nitrobenzyl 3-(2-N-dimethyl-N-oxyamino)ethylsulfinyl-6-ethyl-7-oxo-1-azabicyclo[3.2.0]hept-2-ene-2-carboxylate,

o-nitrobenzyl 3-(2-N-dimethyl-N-oxyamino)ethylsulfinyl-6-isopropyl-7-oxo-1-azabicyclo[3.2.0]hept-2-ene-2-carboxylate,

o-nitrobenzyl 3-(2-N-dimethyl-N-oxyamino)ethylsulfinyl-6-(1-hydroxy)ethyl-7-oxo-1-azabicyclo[3.2.0]hept-2-ene-2-carboxylate,

o-nitrobenzyl 3-(2-N-di-p-nitrobenzylamino)ethylsulfinyl-6-ethyl-7-oxo-1-azabicyclo[3.2.0]hept-2-ene-2-carboxylate,

p-nitrobenzyl 3-(2-N-di-p-nitrobenzylamino)ethylsulfinyl-6-(1-hydroxy)ethyl-7-oxo-1-azabicyclo[3.2.0]hept-2-ene-2-carboxylate,

p-nitrobenzyl 3-(2-N-di-p-nitrobenzyl-N-oxyamino)ethylsulfinyl-6-ethyl-7-oxo-1-azabicyclo[3.2.0]hept-2-ene-2-carboxylate,

p-nitrobenzyl 3-(2-N-di-p-nitrobenzyl-N-oxyamino)ethylsulfinyl-6-(1-hydroxyethyl)-7-oxo-1-azabicyclo[3.2.0]hept-2-ene-2-carboxylate,

p-nitrobenzyl 3-(2-mesylamino)ethylsulfinyl-6-ethyl-7-oxo-1-azabicyclo[3.2.0]hept-2-ene-2-carboxylate,

p-nitrobenzyl 3-(2-mesylamino)-ethylsulfinyl-6-(1-hydroxy)-ethyl-7-oxo-1-azabicyclo[3.2.0]hept-2-ene-2-carboxylate,

o-nitrobenzyl 3-(2-tosylamino)ethylsulfinyl-6-ethyl-7-oxo-1-azabicyclo[3.2.0]hept-2-ene-2-carboxylate, and

p-nitrobenzyl 3-(2-tosylamino)ethylsulfinyl-6-(1-hydroxy)ethyl-7-oxo-1-azabicyclo[3.2.0]hept-2-ene-2-carboxylate.

The reaction of the compound of formula (IV) or its salt with the compound of formula (V) is carried out generally in an inert organic solvent of the type described hereinabove, usually in an inert polar solvent such as N,N-dimethyl formamide (DMF), tetrahydrofuran (THF), dioxane, hexamethylphosphotriamide (HMPA) or glyme, in particular in DMF. The reaction temperature is generally room temperature or below, preferably at low temperature in the range of −30°C to −50°C.

When the compound of formula (V) in which M is hydrogen, i.e. hydrogen sulfide, is used, the reaction is desirably carried out in the presence of the aforesaid base.

The amount of the compound of formula (V), e.g. hydrogen sulfide or sodium hydrosulfide, is not critical. Advantageously, it is used in an amount of generally at least 1.0 mole, preferably 1.1 to 1.5 moles, per mole of the compound of formula (IV).

Thus, the compound of formula (II) can be obtained. This compound can be used in the process of the invention either as such or after it has been isolated and purified in the manner described hereinabove.

The compound of formula (II) obtained as above is a fairly stable compound, but under some conditions, for example, in the presence of trichloroacetonitrile, or under certain oxidizing conditions, it might be dimerized to a compound of the following formula

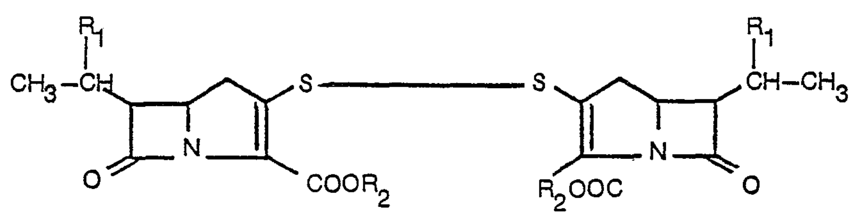

(see Example 29).

The group $—S—CH_2—CH_2—Y$ which is liberated in the reaction of the compound of formula (IV) or its salt with the compound of formula (V) might again react with the resulting compound of formula (II) or its salt under the aforesaid reaction conditions to form a compound of the following formula

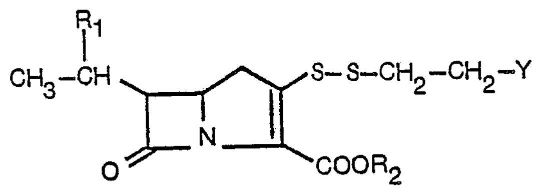

Some of the compounds of formula (IV) used as a starting material in the above reaction are known (see, for example, U.S. Patents Nos. 4,123,547 and 4,150,145), and those which are novel can be produced in the same manner as the production of the aforesaid known compounds. For example, the compound of formula (IV) can be produced by S-oxidation of a compound of the following formula

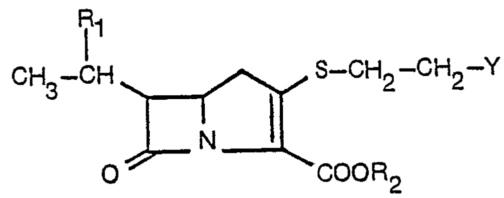

(VII)

wherein $R_1$, $R_2$ and Y are as defined hereinabove, or its salt.

S-oxidation of the compound of formula (VII) can be performed by a method known *per se*, for example by methods frequently utilized in the S-oxidation of sulfur-containing $\beta$-lactam antibiotics of the penicillin or cephalosporin series.

For example, the compound of formula (VII) is reacted with a mild oxidizing agent which does not substantially act on the 7-oxo-1-azabicyclo[3.2.0]hept-2-ene-2-carboxylic acid skeleton, e.g. perbenzoic acid, ozone, phenyl dichloroiodide, hydrogen peroxide, selenium dioxide or sodium metaperiodate. Suitable mild oxidizing agents are organic acids, especially perbenzoic acid and m-chloroperbenzoic acid. Substituted perbenzoic acids such as m-chloroperbenzoic acid are most preferred.

The reaction between the compound of formula (VII) and the oxidizing agent is carried out advantageously in an inert solvent such as methylene chloride, chloroform or carbon tetrachloride at room temperature or at a lower temperature, preferably −30°C to 20°C. The reaction can be completed under these conditions usually within 3 minutes to 3 hours.

The amount of the oxidizing agent used in the oxidation of the compound of formula (VII) can be varied widely depending upon the type of the oxidizing agent, the reaction conditions, etc. Generally, the advantageous amount of the oxidizing agent is 0.3 to 1.8 molar equivalents, preferably 0.7 to 1.3 molar equivalents, per mole of the compound of formula (VII).

After the reaction, the compound of formula (VII). (VII), which is an S-oxidation product, can be isolated and purified by various methods known *per se*. Usually, this compound can be isolated from the reaction mixture by methods frequently utilized in the isolated and purification of carboxyl-containing antibiotics . The compound of formula (VII) can also be used in the aforesaid reaction without isolation and purification.

When the optionally protected amino group at the end of the S-side chain at the 3-position in the compound of formula (VII) or its salt is basic (for example, when it is a free amino group or an amino group which is mono- or di-substituted by an alkyl or aralkyl group), the N atom of the amino group might be simultaneously oxidized by the aforesaid S-oxidation to form an N-oxide

(—N<).
↓
O

However, the reaction of the compound of formula (IV) with the compound of formula (V) proceeds equally even when the terminal amino group is so oxidized.

Compounds of formula (VII) to be subjected to S-oxidation are known, and disclosed, for example, in Japanese Laid-Open Patent Publications Nos. 121702/1978, 30195/1979, 84389/1979, 59295/1979, 54899/1980, 73191/1976, 65293/1977, and 65294/1977, U.S. Patents Nos. 4,123,547 and 4,150,145, and Japanese Patent Applications Nos. 133773/1978 (Laid-Open Publication No. 62088/1980), 94891/1979 (Laid-Open Publication No. 22789/1981, 124712/1979, and 124713/1979). The disclosures of these patent documents are cited in lieu of giving a description of the manufacturing methods for these compounds herein.

Although all chemical formulae in the present application are given in a planar structure for simplicity, it is to be understood that the compounds of formulae (II), (IV) and (VII) used as starting materials in the process of this invention and the compound of formula (I) as the product include steric isomers and optical isomers. Thus, for example, the compound of formula (I) includes the following two steric isomers and a mixture of these.

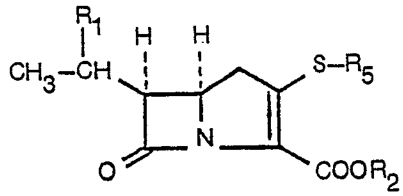

5,6-cis isomer

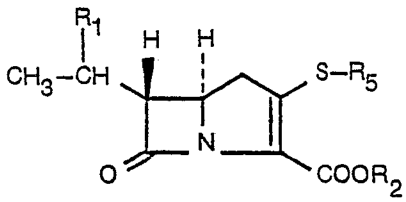

5,6-trans isomer

The compound (I) or its salt obtained by the process of this invention has excellent antimicrobial activity, and can be effectively used as an active ingredient of antimicrobial agents for prevention, therapy and/or treatment of infections by Gram-positive and Gram-negative bacteria not only in man but also in other animals such as mammals, poultry and fish.

The compounds of formula (I-a) and the salts thereof have have antimicrobial activities as stated above, and can be effectively used as an active ingredient of antimicrobial agents for the prevention, treatment and/or medication of infections diseases caused by Gram-positive and Gram-negative bacteria not only in humans but also in other animals such as mammals, poultry, and fish.

The compound of formula (I-a) or its salt may be administered orally, topically or perentally (e.g., intravenously, intramuscularly, intraperitoneally) in a variety of usual pharmaceutical preparations depending on the method of administration. For example, the compound of formula (I-a) or its salt may be formulated with a pharmaceutically acceptable carrier, diluent or the like into solids (e.g., tablets, capsules, powders, granules, sugar-coated tablets, troches, powder sprays, suppositories), semi-solids (e.g., ointments, creams, semisolid capsules), or liquids (e.g., solutions, emulsions, suspension, lotions, syrups, injecting solutions, liquid sprays).

A unit dosage preparation comprising the compound of formula (I-a) or its salt may generally contain from 0.1 to 99% by weight, preferably 10 to 60% by weight, of the active component in any of liquid, semisolid and solid forms. The content of the active ingredient in the pharmaceutical preparation may vary depending upon the form or total amount of the preparation. Usually, it may be from 10 mg to 1000 mg preferably from about 100 mg to about 1000 mg.

A unit dosage form in parenteral administration may be a solution of the compound of formula (I-a) or its salt having a purity of nearly 100% in sterilized water, or a soluble powder of the compound capable of being easily formed into a solution.

In usual oral or parenteral administration, the compound of formula (I-a) may be used in a dose of generally 0.05 to 500 mg/kg per day, preferably 0.5 to 200 mg/kg per day. Needless to say, the dose can be higher or lower than the above specified range depending upon the condition of a patient to be treated.

In addition to using the compound of formula (I-a) as a drug, it can also be added to animal feeds either directly or as a concentrate. It can also be used as an active ingredient in food preservatives or disinfectants.

The following Examples illustrate the present invention in greater detail, Examples 1 to 23 not being Examples of the present invention.

## Example 1

Production of p-nitrobenzyl 3-(2-acetamido)ethylthio-6-ethyl-7-oxo-1-azabicyclo[3.2.0]hept-2-ene-2-carboxylate (PS—5. p-nitrobenzyl ester):—

PS—5 sodium salt (275 mg) was dissolved in 20 mg of dry DMF, and with stirring at room temperature, 197 microliters of triethylamine was added. Ten minutes late, a solution of 279 mg of p-nitrobenzyl bromide in 5 ml of dry dimethylformamide was added at 5°C with stirring. The mixture was stirred at this temperature for 3 hours. After the reaction mixture was poured into a 150 ml of benzene, and washed three times with 100 ml of a 0.1 M phosphate buffer (pH 6.8), and dried over anhydrous sodium sulfate. The solvent was distilled off under reduced pressure, and the residue was chromatographed on a column of silica gel (30 g, 1.8 x 30 cm) using a benzene-acetone (3:1) as an eluent. The eluates were distilled to remove the solvent and thereby to obtain yellow

crystals. Recrystallization of the yellow crystals from benzene afforded 240 mg of yellow needles having a melting point of 163 to 165°C.

$[\alpha]_D^{21}$ + 70.7° (c 1.00, $CHCl_3$).

UV$\lambda_{max}^{CHCl_3}$ ($\varepsilon$): 322 (12800), 269 (12000).

IR$\nu_{max}^{CHCl_3}$ $cm^{-1}$: 3460 (*NH*CO), 1780 (CO of $\beta$-lactam), 1710 (CO of ester), 1675 (NH*CO*), 1530 ($NO_2$), 1350 ($NO_2$).

NMR ($CDCl_3$) $\delta$: 1.04 (3H, 6, J=7Hz, $CH_2CH_3$), 1.84 (2H, m $CH_2CH_3$), 1.96 (3H, s, $NHCOCH_3$), 2.80—3.60 (7H, m, $3XCH_2$, CH), 3.98 (1H, dt, J=9.0Hz, J=3Hz, C—5H), 5.19 (1H, d, J=14Hz, Ar*CH*·H), 5.49 (1H, d, J=14Hz, ArCH·*H*), 5.95 (1H, brs, *NH*), 7.61 (2H, d, J=9Hz, ArH), 8.18 (2H, d, J=9Hz, ArH).

Mass (m/z): 433 ($M^+$), 363 ($M^+$ —EtCH=C=O).

Anal. Calcd. for $C_{20}H_{23}O_6N_3S$:

| | | | |
|---|---|---|---|
| | C, 55.43: | H, 5.35: | N, 9.70 |
| Found: | C, 55.29: | H, 5.24: | N, 9.40 |

Example 2

Production of benzyl 3-(2-acetamido)ethylthio-6-ethyl-7-oxo-1-azabicyclo[3.2.0]hept-2-ene-2-carboxylate (PS—5) ·benzyl ester):—

Prepared in the same way as in Example 1.

$[\alpha]_D^{21}$ + 19.97° (c 0.33, $CHCl_3$).

UV$\lambda_{max}^{MeOH}$ nm ($\varepsilon$): 318 (11000).

IR$\nu_{max}^{CHCl_3}$ $cm^{-1}$: 3430 (CO*NH*), 1770 (CO of $\beta$-lactam), 1690 (CO of ester), 1665 (*CO*NH).

NMR ($CDCl_3$) $\delta$: 1.04 (3H, t, J=7Hz, $CH_2CH_3$), 1.82 (2H, m, $CH_2CH_3$), 1.96 (3H, s, $COCH_3$), 2.80—3.60 (7H, m, $3XCH_2$, CH), 3.94 (1H, dt, J=9.0Hz, J=3Hz, C—5, *H*), 5.21 (1H, d, J=12Hz, Ar*CH*·H), 5.38 (1H, d, J=12Hz, ArCH·*H*), 5.90—6.10 (1H, brs, N*H*), 7.34 (5H, distored, s, ArH).

Mass (m/z): 388 ($M^+$).

Example 3

Production of benzhydryl 3-(2-acetamido)ethylthio-6-ethyl-7-oxo-1-azabicyclo[3.2.0]hept-2-ene-2-carboxylate (PS—5. benzhydryl ester):—

Prepared in the same way as in Example 1.

$[\alpha]_D^{21}$ + 2.55° (*C*=0.33, $CHCl_3$).

UV$\lambda_{max}^{MeOH}$ nm ($\varepsilon$): 320 (11200).

IR$\nu_{max}^{CHCl_3}$ $cm^{-1}$: 3450 (—CO*NH*—), 1775 (CO of $\beta$-lactam), 1675 (CO of ester).

NMR ($CDCl_3$) $\delta$: 1.06 (3H, t, J=8Hz, $CH_3CH_2$—), 1.94 (3H, s, $CH_3CONH$—), 3.94 (1H, dt, J=9Hz, J=3Hz, C—5H), 5.92 (1H, brs, NH), 6.90 (1H, s, —$CHAr_2$), 7.32 (10H, s, —$CHAr_2$).

Mass (m/z): 464 ($M^+$), 394 ($M^+$ —EtCH=C=O).

Example 4

Production of methyl 3-(2-acetamido)ethylthio-6-ethyl-7-oxo-1-azabicyclo[3.2.0]hept-2-ene-2-carboxylate (PS—5. methyl ester):—

Prepared in the same way as in Example 1.

$[\alpha]_D^{21}$ + 72.23° (C=0.36, $CHCl_3$).

UV$\lambda_{max}^{MeOH}$ nm ($\varepsilon$): 315 (10300).

IR$\nu_{max}^{CHCl_3}$ $cm^{-1}$: 3450 (—CO*N*H), 1775 (CO of $\beta$-lactam), 1675 (CO of ester).

NMR ($CDCl_3$) $\delta$: 1.06 (3H, t, J=8Hz, —CH—$CH_3$), 1.70—2.06 (2H, m, —$CH_2CH_3$), 1.99 (3H, s, —$NHCOCH_3$), 2.90—3.56 (7H, m, 3X—$CH_2$—, —CH—), 3.83 (3H, s, —$CO_2CH_3$), 3.96 (1H, dt, J=8Hz, J=2Hz, C—5*H*), 6.20 (1H, brs, —*NH*).

Mass (m/z): 312 ($M^+$), 242 ($M^+$ —EtCH=C=O).

Example 5

Production of pivaloyloxymethyl 3-(2-acetamido)ethylthio-6-ethyl-7-oxo-1-azabicyclo[3.2.0]hept-2-ene-2-carboxylate (PS—5. pivaloyloxymethyl ester):—

Prepared in the same way as in Example 1.

$[\alpha]_D^{24}$ + 68.0° (C=10, THF).

UV$\lambda_{max}^{THF}$ nm ($\varepsilon$): 323.5 (11200).

IR$\nu_{max}^{CHCl_3}$ $cm^{-1}$: 1772 (CO of $\beta$-lactam), 1750, 1718 (CO of ester)

NMR ($CDCl_2$) $\delta$: 1.03 (3H, t, J=7.5Hz, $CH_2CH_3$)

$$1.22\ (9H,\ s,\ CH_3{-}\overset{\displaystyle CH_3}{\underset{}{\overset{|}{C}}}{-}CH_3)$$

1.65—1.95 (2H, m, $CH_2CH_3$)

1.98 (3H, s, $COCH_3$)

2.70—3.70 (7H, m, C—4$H_2$, C—6*H*, S—$CH_2CH_2$NH)

3.92 (1H, dt, J=3.0Hz, J=9.0Hz, C—5*H*)

5.83 (1H, d, J=12.0Hz, O—C*H*H—O)
5.90 (1H, d, J=12.0Hz, O—CH*H*—O)
6.15 (1H, br, N*H*)
Mass (m/z): 412 ($M^+$).

Example 6

Production of p-nitrobenzyl 3-(2-acetamido)ethylthio-6-isopropyl-7-oxo-1-azabicyclo[3.2.0]hept-2-ene-2-carboxylate (PS—6, p-nitrobenzyl ester):—

Prepared in the same way as in Example 1.

UV$\lambda_{max}^{THF}$ nm (δ): 322 (δ=12500), 269 (11800)

IR$\nu_{max}^{CHCl_3}$ $cm^{-1}$: 1780 (CO of β-lactam), 1708 (CO of ester).

NMR ($CDCl_3$) δ: 1.02 (3H, d, J=7.0Hz, 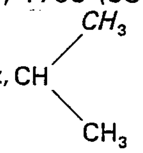 ),

1.05 (3H, d, J=7.0Hz, 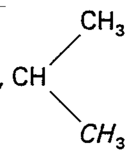 ),

1.90—2.25 (1H, m, 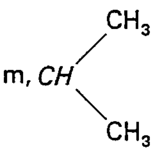 ),

1.98 (3H, S, $COCH_3$), 2.80—3.60 (7H, m, C—4H, C—6H, 2 × $CH_2$), 4.02 (1H, dt, J=9.0Hz, J=3.0Hz, C—5H), 5.20 (1H, d, J=14.0Hz, ArC*H*H), 5.48 (1H, d, J=14.0Hz, ArCH*H*), 6.00 (1H, m, NH), 7.62 (2H, d, J=9.0Hz, ArH), 8.18 (2H, d, J=9.0Hz, Ar*H*).

Example 7

Benzyl 3-(2-acetamido)ethylthio-6-isopropyl-7-oxo-1-azabicyclo[3.2.0]hept-2-ene-2-carboxylate (PS—6. benzyl ester):—

Prepared in the same way as in Example 1.

UV$\lambda_{max}^{MeOH}$ 318 nm.

IR$\nu_{max}^{CHCl_3}$ $cm^{-1}$: 3400 (N*H*—CO), 1765 (CO of β-lactam), 1665 (NH*CO*).

NMR ($CDCl_3$) ε: 1.07 (6H, t, J=8Hz, —CH·($CH_3$)$_2$), 2.00 (3H, s, —NHCOC$H_3$), 4.05 (1H, dt, C—5H), 5.31 (1H, d, J=12Hz, —*CH*, HAr), 5.47 (1H, d, J=12Hz, —CH, *H*Ar), 6.07 (1H, brs, NH—), 7.46 (5H, s, Ar-H).

Mass (m/z): 402 ($M^+$), 318 ($M^+$ —i·Pr·CH=C=O).

Example 8

Production of p-nitrobenzyl 5,6-trans-3-(2-acetamidoethylthio)-6-(1-hydroxyethyl)-7-oxo-1-azabicyclo[3.2.0]hept-2-ene-2-carboxylate (PS—3A. p-nitrobenzyl) ester:—

Prepared in the same way as in Example 1.

UV$\lambda_{max}^{THF}$ nm (ε): 322 (13500), 270 (11500).

IR$\nu_{max}^{KBr}$ $cm^{-1}$: 3475, 3300 (OH, NH), 1765 (CO of β-lactam), 1700 (CO of ester), 1650 (CO of amide).

NMR ($CDCl_3$) δ: 1.37 (3H, d, J=6.5Hz, —CHOH—$CH_3$), 1.96 (3H, S, —COC$H_3$), 2.50—3.65 (7H, m, C6—*H*, C4—$H_2$, —S—$CH_2$—$CH_2$—NH—), 3.80—4.40 (2H, m, —*CH*OH—$CH_3$, C5—*H*), 5.21 (1H, d, J=14.0Hz, —C*H*H—Ar), 5.45 (1H, d, J=14.0Hz, —CH*H*—Ar), 7.59 (2H, d, J=8.4Hz, 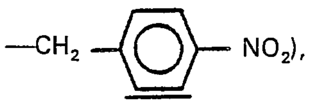 ), 8.16 (2H, d, J=8.4Hz, 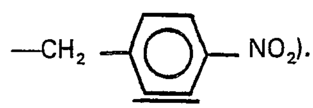 ).

Mass (m/z): 363 ($M^+$ —86).

Example 9

Production of p-nitrobenzyl 5,6-cis-3-(2-acetamidoethylthio)-6-(1-hydroxyethyl)-7-oxo-1-azabicyclo[3.2.0]hept-2-ene-2-carboxylate (PS—3B ♎ p-nitrobenzyl ester):—

Prepared in the same way as in Example 1.

UV$\lambda_{max}^{THF}$ nm (ε): 320 (11500), 270 (9700).

$IR\nu_{max}^{KBr}$ $cm^{-1}$: 3410 (OH, NH), 1780 (CO of β-lactam), 1700 (CO of ester), 1660 (CO of amide).

NMR ($CDCl_3$) δ: 1.41 (3H, d, J=6.4Hz, —CHOH—$CH_3$), 1.96 (3H, s, —CO—$CH_3$), 2.83—3.91 (7H, m, C—4H, C—6H, —S$CH_2CH_2$NH—), 4.00—4.50 (2H, m, C—5*H*, —*C*HOH—), 5.21 (1H, d, J=14.0Hz, —*C*HH—Ar), 5.45 (1H, d, J=14.0Hz, —CH*H*—Ar), 7.60 (2H, d, J=8.41Hz, —$CH_2$—*Ar*), 8.17 (2H, d, d, J=8.4Hz, —$CH_2$—*Ar*).

Mass (m/z): 363 ($M^+$ —$CH_3$CHOH·CH=C=O).

## Example 10

Production of thienamycin:

In accordance with the method described in Journal of Antibiotic *32*, Nos. 11—12, 1979, *Streptomyces cattleya* (NRRL 8057) was inoculated in an ISP-2- agar culture medium to cause full adhesion of spores. A platinum loopful of the resulting slant culture was inoculated in a 500 ml Erlenmeyer flask containing 100 ml of an SE—4 seed culture medium consisting of 0.3% of beef extract (a product of Difco Laboratories), 0.5% of bactotryptone (a product of Difco Laboratories), 0.5% of soybean meal, 0.1% of glucose, 2.4% of soluble starch, 0.5% of yeast extract and 0.4% of $CaCO_3$ and having the pH adjusted to 7.5, and cultivated with shaking at 28°C.

Two milliliters of the seed culture was inoculated in 200 Erlenmeyer flasks (capacity 500 ml) each containing 100 ml of a culture medium consisting of 2.5% of glucose, 1.0% of corn steep liquor, 0.5% of yeast extract, 0.5% of Pharmamedia, 0.3% of $CaCO_3$ and 0.0013% of $CoCl_2 \cdot 6H_2O$ and having the pH adjusted to 7.3, and cultivated with shaking at 28°C for 5 days.

The culture broth was put into ice water to cool it rapidly, and 3% of Highflow Supercell (a filtration aid made by Jones Manville Co.) was added. It was suction-filtered to give 21 liters of a filtrate. About 48 μg/ml of thienamycin was found to be contained in the filtrate. Operations subsequent to this were all performed at a low temperature of 6°C.

The pH of the filtrate was carefully lowered to 4.5, and adsorbed to a column (7 x 90 cm) of Dowex 50 x 4 (Na-form 20—50 mesh) equilibrated by water and eluted with 2% pyridine. The eluate was concentrated under reduced pressure, and its pH was adjusted to 7.3. Then, it was charged onto a column (3 x 50 cm) of Dowex 1 x 2 Cl-form 50—100 mesh equilibrated with water, and eluted with water. The eluate was lyophilized.

A column (2.5 x 40 cm) of Dowex 50 x 8 lutidine-form 200—400 mesh was equilibrated with a 0.25 M lutidine acetate buffer (pH 6.3), and a solution of the lyophilized product in 5 ml of 0.1 M lutidine acetate (pH 6.3) was charged onto the column. The column was eluted with a buffer (pH 6.3) of 0.25 M lutidine acetate. Throughout the entire process, active fractions were detected by bioassay using *Comamonas terrigena* IFO 12685 as an assay microorganism and by coloration in ninhydrin and Ehrlich reactions.

The active fractions were collected, and lyophilized. The lyophilized product was dissolved in 2 ml of a phosphate buffer (0.01 M, pH 7.0). The pH of the solution was adjusted to 7.0, and the solution was charged onto a column (1.1 x 90 cm) of Biogel P—2 (a product of Bio-Rad Co.) equilibrated with the aforesaid buffer and developed by the same buffer. Active fractions were collected and adsorbed to a column (1.1 x 40 cm) filled with Diaion HP—20 AG (a product of Mitsubishi Chemical Industries, Ltd.), followed by elution with water. The active fractions were lyophilized to give 14 mg of a white lyophilized standard product.

The purity of the product, measured on the basis of $E_{1}^{1\%}$ cm 290, was 40 to 70% although varying from lot to lot.

## Example 11

Production of p-nitrobenzyl (5R, 6S)3-(2-acetamido)ethylthio-6-[(1R)1-hydroxy]ethyl-7-oxo-1-azabicyclo[3.2.0]hept-2-ene-2-carboxylate (N-acetylthienamycin p-nitrobenzyl ester):—

Thienamycin (18.8 mg) was dissolved in 4.0 ml of dry dimethyl formamide to form an emulsion. The emulsion was cooled with ice water, and with stirring, 2.0 ml of acetic anhydride as distilled was added. The mixture was stirred at the same temperature for 40 minutes.

The reaction mixture was washed with 25 ml of hexane ten times, and when dimethyl formamide and acetic anhydride were almost exhausted, 0.5 ml of ether was added. The mixture was then washed with 25 ml of hexane twice.

The residue was dried under reduced pressure for 3 minutes, and dissolved in 4.0 ml of dry dimethyl formamide. The solution was cooled with ice water, and with stirring, 144 microliters of triethylamine was added. Then a solution of 149 mg of p-nitrobenzyl bromide in 1.5 ml of dimethyl formamide was gradually added dropwise to the mixture. After the addition, the temperature was raised to room temperature, and the reaction was performed for 2 hours. The reaction mixture was poured ino 40 ml of ethyl acetate, cooled, and washed with 20 ml of 0.1 M phosphate buffer (pH 6.86) three times. The product was dried over anhydrous sodium sulfate, and concentrated to dryness. Dichloromethane (1 ml) was added, and the precipitated solid was collected by filtration. The solid was washed with 2 to 3 ml of dichloromethane, and dried under reduced pressure to give 7.2 mg of the desired product as a white solid. The mother liquor was concentrated under reduced pressure, and chromatographed on a column (1.0 x 8.0 cm) of silica gel (4 gr) with a developing solvent consisting of

a 1:2 mixture of benzene and acetone. Fractions containing the desired product which had an Rf value of 0.10 (benzene/acetone=1/1) by TLC were collected and concentrated to dryness to give 4.0 mg of the desired product as a white solid. The total amount of the product was 11.2 mg (yield 36.1%).

$[\alpha]_D^{23}$ + 50.1° (C 0.2, MeOH).

$UV\lambda_{max}^{MeOH}$ nm ($\varepsilon$): 319.2 (14800), 268.5 (13100).

$IR\nu_{max}^{KBr}$ $cm^{-1}$: 1770 (C=O of $\beta$-lactam), 1692 (C=O of ester).

NMR (DMSO-$d_6$) $\delta$:

1.15 (3H, J=7Hz, —CH(OH)—$CH_3$).
1.82 (3H, S, —NHCO—$CH_3$),
2.80—3.50 (7H, m, —S—$CH_2$—$CH_2$—NHAc, C—4$H_2$, C—6H),
3.60—4.30 (2H, m, —CH(OH)—$CH_3$, C—5H),
5.24 (1H, d, J=14.5Hz, —CH·H—Ar).
5.47 (1H, d, J=14.5Hz, —CH·H—Ar),
7.72 (2H, d, J=9Hz, —ArH),
8.27 (2H, d, J=9Hz, —ArH),

## Example 12

Production of p-nitrobenzyl (5R, 6S)6-[(1R)1-hydroxy)]ethyl-3-(2-p-nitrobenzyloxycarbonyl-amino)ethylthio-7-oxo-1-azabicyclo[3.2.0]hept-2-ene-2-carboxylate (N-p-nitrobenzyloxycarbonyl thienamycin):—

With ice cooling, 4.62 mg (0.0055 millimole) of sodium hydrogen carbonate and 3 ml of dioxane were added to a solution of 15 mg (0.055 millimole) of thienamycin in 3 ml of distilled water. Then, a solution of 17.7 mg (0.082 millimole) of p-nitrobenzyl chloroformate in 0.5 ml of dioxane was added dropwise with stirring. The mixture was stirred for 10 minutes with ice cooling. The reaction mixture was washed with 2 ml of ether twice, and 0.1 M sulfuric acid was added. The pH of the aqueous layer was adjusted to 2.3, and it was extracted with 3 ml of ethyl acetate three times. The organic layers were combined, and washed with a cold saturated aqueous solution of sodium chloride, dried over anhydrous sodium sulfate, and concentrated under reduced pressure to a volume of about 3 ml. Immediately then, 5 ml of anhydrous dimethyl formamide and 16.8 microliters (0.121 millimole) of triethylamine was added. With ice cooling and stirring, a solution of 13.1 mg (0.0605 millimole) of p-nitrobenzyl bromide in 1 ml of dimethyl formamide was added dropwise. Then, the temperature was gradually raised to room temperature, and the mixture was stirred for 2 hours.

The reaction mixture was poured into 50 ml of ethyl acetate, washed with a cold 0.1 M phosphate buffer (pH 6.8) (10 ml × 4). The organic layer was dried over anhydrous sodium sulfate and concentrated. The residue was washed with ether to give 12 mg of the desired product as an insoluble substance.

$UV\lambda_{max}^{MeOH}$ ($\varepsilon$): 268 (22100), 319 (13600).

$IR\nu_{max}^{KBr}$ $cm^{-1}$: 1772 (CO of $\beta$-lactam), 1691 (CO of ester), 3400 (OH, NH).

NMR (DMSO—$d_6$) $\delta$: 1.15 (3H, d, J=6.1Hz, —CH·$CH_3$).

2.7—3.6 (7H, m, —S·$CH_2CH_2$—NH—, C—4$H_2$, C—6H).
3.7—4.3 (2H, m, C—5H, —CH—OH).
5.22 (2H, S, —NH—$CO_2$—$CH_2$Ar).
5.27 (1H, d, J=14.5Hz, —CH—HAr).
5.48 (1H, d, J=14.5Hz, —CH—HAr).
7.5—7.8 (4H, m, ArH).
8.1—8.4 (4H, m, ArH).

## Example 13

Production of p-nitrobenzyl 3-(2-acetamido) ethylsulfinyl-6-ethyl-7-oxo-1-azabicyclo[3.2.0]hept-2-ene-2-carboxylate:—

240 mg of p-nitrobenzyl 3-(2-acetamido)ethylthio-6-ethyl-7-oxo-1-azabicyclo[3.2.0]hept-2-ene-2-carboxylate was dried, and dissolved in 25 ml of methylene chloride. The solution was cooled to —30°C. A solution of 104.9 mg of m-chloroperbenzoic acid in 12 ml of dry methylene chloride was added to the above solution with stirring, and the reaction was performed at this temperature for 40 minutes. After the reaction, the reaction mixture was poured into an ice-cooled solution of 0.09 ml of triethylamine in 100 ml of ethyl acetate, and stirred for 5 minutes with ice cooling. Then, 100 ml of ethyl acetate was added, and the mixture was transferred to a separating funnel and washed with an ice-cooled 0.1 M phosphate buffer (pH 6.86) four times. The solvent layer was dried over anhydrous sodium sulfate, and benzene was added. The mixture was concentrated under reduced pressure to a volume of 1 ml. The concentrate was passed through a silica gel column (18 g; 42 ml, 2.0 × 13.4 cm; the same silica gel as mentioned hereinabove) wetted with benzene-acetone (1:1), and developed with

about 50 ml of the same solvent mixture. The eluate contained 14.1 mg of the starting material. When the column was developed further with about 60 mg of benzene-acetone (1:2) and then 70 ml of benzene-acetone (1:3) to give the title compound (amount 211 mg; yield 89%). On a silica gel TLC plate (the same TLC plate as described hereinabove), this product showed an Rf value of 0.36 with benzene-acetone (1:2).

$[\alpha]_D^{21}$ + 18.0° (c 1.00 $CHCl_3$).

UV$\lambda_{max}^{CHCl_3}$ nm ($\varepsilon$): 267 (13100), 310 (7800).

IR$\nu_{max}^{CHCl_3}$ cm$^{-1}$: 3460 (*N*HCO), 1785 (CO of $\beta$-lactam), 1720 (CO of ester), 1680 (NH*CO*).

NMR ($CDCl_3$) $\delta$:

1.08 (3H, t, J=7.0Hz, $CH_2CH_3$), 1.84 (2H, m, $CH_2CH_3$),

2.00 (3H, S, NHCO$CH_3$), 2.90—3.92 (7H, m, 3X$CH_2$, CH), 3.92—4.23 (1H, m, CH),

5.22 (1H, d, J=14.0Hz, ArC*H*H), 5.48 (1H, d, J=14.0Hz, ArCH*H*), 6.40 (1H, brs, N*H*),

7.53—7.72 (2H, d, J=9.0Hz, Ar*H*),

8.12—8.28 (2H, d, J=9.0Hz, ArH).

Mass (m/z): 449 ($M^+$) (FD Mass).

## Example 14

Benzyl 3-(2-acetamido)ethylsulfinyl-6-ethyl-7-oxo-1-azabicyclo[3.2.0]hept-2-ene-2-carboxylate:—

Prepared in the same way as in Example 13.

$[\alpha]_D^{21}$ – 43.96 (c 1.00, MeOH).

UV$\lambda_{max}^{MeOH}$ nm ($\varepsilon$): 307 (4400).

IR$\nu_{max}^{CHCl_3}$ cm$^{-1}$: 3400 (N*H*CO), 1790 (CO of $\beta$-lactam), 1710 (CO of ester), 1670 (NH*CO*).

NMR ($CDCl_3$) $\delta$: 1.04 (3H, t, J=7.0Hz, $CH_2CH_3$), 1.86 (2H, m, $CH_2CH_3$), 1.94 (3H, s, CO$CH_3$), 2.90—3.84 (7H, m, 3X$CH_2$, C*H*), 4.12 (1H, m, C—5*H*), 5.26 (2H, s, Ar$CH_2$), 6.40 (1H, m, NH), 7.36 (5H, s, Ar*H*).

Mass (m/z): 404 ($M^+$).

## Example 15

Production of benzhydryl 3-(2-acetamido)ethyl-sulfinyl-6-ethyl-7-oxo-1-azabicyclo[3.2.0]hept-2-ene-2-carboxylate:—

Prepared in the same way as in Example 13.

$[\alpha]_D^{21}$ – 64.04° (*c* 0.28, $CHCl_2$).

UV$\lambda_{max}^{MeOH}$ nm ($\varepsilon$): 308 (5600).

IR$\nu_{max}^{CHCl_3}$ cm$^{-1}$: 3450 (*N*HCO—), 1790 (CO of $\beta$-lactam), 1710 (CO of ester), 1670 (NH*CO*).

NMR ($CDCl_3$) $\delta$:

1.08 (3H, t, J=8Hz, —$CH_2$—$CH_3$),

1.92 (3H, s, —NHCO$CH_3$),

4.08 (1H, dt, J=9Hz, J=3Hz, C—5H),

6.34 (1H, br, N*H*), 6.87 (1H, s, —*CH*$Ar_2$),

7.32 (10H, s-like, —CH$Ar_2$),

Mass (m/z): 480 ($M^+$).

## Example 16

Production of methyl 3-(2-acetamido)ethyl-sulfinyl-6-ethyl-7-oxo-1-azabicyclo[3.2.0]hept-2-ene-2-carboxylate:—

Prepared in the same way as in Example 13.

$[\alpha]_D^{21}$ – 74.23° (c 0.25, $CHCl_3$).

UV$\lambda_{max}^{MeOH}$ nm ($\delta$): 303 (5200).

IR$\nu_{max}^{CHCl_3}$ cm$^{-1}$: 3450 (*N*HCO), 1790 (CO of $\beta$-lactam), 1720 (CO of ester), 1670 (NH*CO*).

NMR ($CDCl_3$) $\delta$:

1.07 (3H, t, J=8Hz, —$CH_2$—$CH_3$),

1.70—2.00 (2H, m, —$CH_2$—$CH_3$),

2.00 (3H, s, —NHCO$CH_3$), 2.90—3.90 (7H, m, 3X—$CH_2$—, —CH—), 3.89 (3H, s, —$CO_2CH_3$),

4.00—4.20 (1H, m, C—5H), 6.60 (1H, brs, —NH).

Mass (m/z): 328 ($M^+$) (FD Mass).

## Example 17

Production of pivaloyloxymethyl 3-(2-acetamino)ethylsulfinyl-6-ethyl-7-oxo-1-azabicyclo[3.2.0]hept-2-ene-2-carboxylate (the sulfoxide of PS—5·pivaloyloxymethyl ester):—

184 mg (0.45 millimole) of the PS—5·pivaloyloxymethyl ester prepared in Example 5 was dissolved in 10 ml of methylene chloride, and cooled to –30°C. A solution of 85.0 mg (0.49 millimole) of m-chloroperbenzoic acid in 10 ml of methylene chloride was added dropwise, and the reaction was performed at the same temperature for 30 minutes. The reaction mixture was poured into 100 ml of methylene chloride, washed with a saturated aqueous solution of sodium carbonate, and dried over

anhydrous sodium sulfate. Benzene was added to the organic layer, and the mixture was concentrated under reduced pressure. The concentrate was adsorbed to a column of silica gel (25 ml) and eluted with benzene-acetone (1:1), (1:3), and (1:5) respectively. Those eluates which had an UV absorption at Rf=0.2 in silica gel TLC developed with benzene-acetone (1:2) were dried under reduced pressure to give 147 mg of the title compound.

$[\alpha]_D^{24}$ + 15.8° (C=1.0, THF).
$UV\lambda_{max}^{THF}$ nm ($\varepsilon$): 317 (5900)
$IR\nu_{max}^{CHCl_3}$ $cm^{-1}$: 1788 (CO of $\beta$-lactam), 1755, 1725 (CO of ester),
NMR ($CDCl_3$) $\delta$:
1.06 (3H, t, J=7.5Hz, $CH_2CH_3$),
1.27 (9H, s, 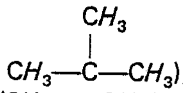),
1.70—2.05 (2H, m, $CH_2CH_3$),
2.02 (3H, s, $COCH_3$),
2.90—4.30 (8H, m, C—4*H*, C—5*H*, C—6*H*, 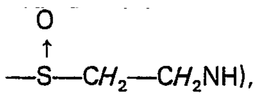),
5.80—6.05 (2H, m, $COOCH_2OCO$),
6.55 (1H, m, N*H*).

## Example 18

Production of p-nitrobenzyl 3-(2-acetamido)ethylsulfinyl-6-isopropyl-7-oxo-1-azabicyclo[3.2.0]hept-2-ene-2-carboxylate (the sulfoxide of PS—6·p-nitrobenzyl ester):—

Prepared in the same way as in Example 13.
$UV\lambda_{max}^{CHCl_3}$ nm ($\varepsilon$): 267 (13000), 310 (7800).
$IR\nu_{max}^{CHCl_3}$ $cm^{-1}$: 1792 (CO of $\beta$-lactam), 1720 (ester).
NMR ($CDCl_3$) $\delta$:
1.00 (3H, d, J=7.0Hz, 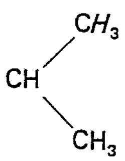),
1.03 (3H, d, J=7.0Hz, 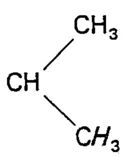),
1.90—2.20 (4H, m, 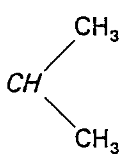, $COCH_3$),
2.90—4.25 (8H, m, C—4H, C—5H, C—6H, 2 × $CH_2$),
5.22 (1H, d, J=14.0Hz, ArC*H*H),
5.44 (1H, d, J=14.0Hz, ArCH*H*),
6.45 (1H, m, NH),
7.56 (2H, d, J=9.0Hz, ArH),
8.18 (2H, d, J=9.0Hz, ArH).

## Example 19

Production of benzyl 3-(2-acetamido)ethylsulfinyl-6-isopropyl-7-oxo-1-azabicyclo[3.2.0]hept-2-ene-2-carboxylate:—

Prepared in the same way as in Example 13.
$UV\lambda_{max}^{MeOH}$ : 307 nm.
$IR\nu_{max}^{CHCl_3}$ $cm^{-1}$: 3400 (—N*H*CO), 1780 (CO of $\beta$-lactam), 1710 (CO of ester), 1670 (NH*CO*).
NMR ($CDCl_2$) $\delta$:
1.07 (6H, t, J=7Hz, —$CH(CH_3)_2$),
1.98 (3H, s, —$NHCOCH_3$),
4.12 (1H, m, C—5H),
5.29 (2H, s, —$CH_2$—Ar),
6.47 (1H, br, —N*H*),
7.37 (5H, s, -like, ArH).
Mass (m/z): 418 ($M^+$) (FD Mass).

Example 20

Production of p-nitrobenzyl 5,6-trans-3-(2-acetamido)ethylsulfinyl-6-(1-hydroxy)ethyl-7-oxo-1-azabicyclo[3.2.0]hept-2-ene-2-carboxylate (the sulfoxide of PS—3A·p-nitrobenzyl ester):—

Prepared in the same way as in Example 13.

UV$\lambda_{max}^{THF}$ nm ($\varepsilon$): 323 (sh, 3300), 270 (8900).

IR$\nu_{max}^{CHCl_3}$ cm$^{-1}$: 3450 (—OH), 1790 (CO of $\beta$-lactam), 1720 (CO of ester), 1680 (CO of amide).

NMR ($CDCl_3$) $\delta$:

1.41 (3H, d, J=6.0Hz, —CHOH—$CH_3$), 1.97 (3H, s, —CO—$CH_3$), 3.00—3.90 (7H, m, C—6H, C—4$H_2$, S—$CH_2$—$CH_2$NH),
4.00—4.55 (2H, m, C—5H, C—7H),
5.25 (1H, d, J=14Hz, CHH—Ar),
5.42 (1H, d, J=14Hz, CHH—Ar),
6.10—6.50 (1H, m, —NH—),
7.58 (2H, d, J=9.0Hz, —ArH),
8.19 (2H, d, J=9.0Hz, —ArH).

Example 21

p-Nitrobenzyl 5,6-cis-3-(2-acetamido-ethylsulfinyl-6-(1-hydroxy)ethyl-7-oxo-1-azabicyclo[3.2.0]hept-2-ene-2-carboxylate (the sulfoxide of PS—3B·p-nitrobenzyl ester):—

Prepared in the same way as in Example 13.

UV$\lambda_{max}^{THF}$ nm ($\varepsilon$): 324 (sh, 4500), 270 (14700).

IR$\nu_{max}^{CHCl_3}$ cm$^{-1}$: 3380 (—OH, NH), 1780 (CO of $\beta$-lactam), 1710 (CO of ester), 1665 (CO of amide).

NMR ($CDCl_3$) $\delta$:

1.41 (3H, d, J=6.0Hz, CHOH—$CH_3$),
1.96 (3H, S, CO—$CH_3$),
2.90—3.95 (7H, m, C—6H, C—4$H_2$, S$CH_2CH_2$N),
5.24 (1H, d, J=14.0Hz, CHHAr), 5.41 (1H, d, J=14.0Hz, CHHAr), 7.58 (2H, d, J=8.4Hz, ArH),
8.19 (2H, d, J=8.4Hz, ArH).

Example 22

Production of p-nitrobenzyl (5R, 6S)-3-(2-acetamido)-ethylsulfinyl-6-[(1R)-1-hydroxy]ethyl-7-oxo-1-azabicyclo[3.2.0]hept-2-ene-2-carboxylate (N-acetylthienamycin.p-nitrobenzyl ester.sulfoxide):—

28.8 mg of N-acetylthienamycin p-nitrobenzyl ester was dissolved in 10 ml of dry tetrahydrofuran which had just been distilled. The solution was cooled to −30°C, and a solution of 14.4 mg of m-chloroperbenzoic acid in 1.44 ml of dry tetrahydrofuran was added dropwise over the course of 5 minutes with stirring. After the addition, the mixture was stirred at the same temperature for 30 minutes, poured into 100 ml of ethyl acetate, and then washed with a cooled saturated aqueous solution of sodium hydrogen carbonate (50 ml × 2) and a saturated aqueous solution of sodium chloride (50 ml × 1). The washed product was dried over anhydrous sodium sulfate, and concentrated under reduced pressure. The resulting residue (32.5 mg) was chromatographed on a column (0.5 × 5 cm) of silica gel (0.5 g) using benzene-acetone (1:3) as an eluent. Fractions which contained the desired product (Rf=0.07 in TLC with benzene-acetone (1:3)) were collected and concentrated to dryness to afford 16.2 mg (yield 54.4%) of the desired product.

UV$\lambda_{max}^{MeOH}$ nm($\varepsilon$): 319 (3600, sh), 269 (10200).

IR$_{nmax}^{KBr}$ cm$^{-1}$: 1780 (c=O of $\beta$-lactam),
1700 (C=O of ester).

NMR (DMSO-$d_6$) $\delta$:

1.14 (3H, d, J=6.5Hz, —CH(OH)—$CH_3$),
1.78 (3H, s, —NHCO$CH_3$),
2.80—3.65 (7H, m, —S—$CH_2$—$CH_2$—NHAc, C—4$H_2$, C—6H),
3.70—4.35 (2H, m, —CH(OH)—$CH_3$, C—5H),
5.40(2H, s, —$CH_2$—Ar),
7.71 (2H, d, J=9Hz, Ar),
8.25 (2H, d, J=9Hz, Ar).

Example 23

Production of p-nitrobenzyl (5R, 6S)-6-[(1R)1-hydroxy]-ethyl-3-(2-p-nitrobenzyloxycarbonylamino)-ethylsulfinyl-7-oxo-1-azabicyclo[3.2.0]hept-2-ene-2-carboxylate (N-p-nitrobenzyloxycarbonyl-thienamycin.p-nitrobenzyl ester.sulfoxide):

A solution of 10 mg (0.017 millimole) of N-(p-nitrobenzyloxycarbonyl)thienamycin p-nitrobenzyl ester in 2 ml of anhydrous tetrahydrofuran was cooled to −20°C, and with stirring, a solution of 7.3 mg (0.0338 millimole) of m-chloroperbenzoic acid in 0.5 ml of tetrahydrofuran was added dropwise in an atmosphere of nitrogen. The mixture was stirred at the same temperature for 30 minutes, diluted with

10 ml of ethyl acetate, washed with a cold saturated solution of sodium hydrogen carbonate (2 ml x 2) and a saturated aqueous solution of sodium chloride (2 ml=2), dried over anhydrous sodium sulfate, and concentrated to give a solid. The solid was washed with ethyl acetate to give 5 mg of the desired product as an insoluble substance.

UV$\lambda_{max}^{MeOH}$: 268 (21100), 310 (7700).
IR$\nu_{max}^{KBr}$: 1781 (CO of $\beta$-lactam), 1695 (CO of ester).
NMR ($CDCl_3$) $\delta$:
1.36 (3H, d, J=6Hz, >CH—$CH_3$),
3.0—3.9 (7H, m, C—6*H*, C—4$H_2$, —S—$CH_2$—$CH_2$—HH—),
4.0—4.6 (2H, m C—5*H*, >*CH*—OH),
5.0—5.7 (4H, m, —O—$CH_2$—Ar x 2),
7.5—7.8 (4H, m, ArH x 4),
8.1—8.4 (4H, m, ArH x 4).

## Example 24

Production of p-nitrobenzyl 6-ethyl-3-mercapto-7-oxo-1-azabicyclo[3.2.0]hept-2-ene-2-carboxylate:

49.5 mg of PS-5-p-nitrobenzyl ester.sulfoxide was dissolved in 15 ml of dry dimethyl formamide, and the solution was cooled to −40°C. With stirring, 0.94 ml of a dimethyl formamide solution of sodium hydrosulfide ($NaSH.XH_2O$ abt 70%) (10 mg/ml) was gradually added. The mixture was stirred at the same temperature for 30 minutes. The reaction mixture was poured into 70 ml of benzene, and washed with a cold 0.1M phosphate buffer (pH 6.86) (30 ml x 3). The benzene layer was dried over anhydrous sodium sulfate, concentrated under reduced pressure to a volume of about 0.3 ml, charged onto a column (1.2 x 90 cm) of Biobeads S—X3 and developed with benzene. Fractions which showed an UV absorption at Rf=0.55 in silica gel TLC with benzene-acetone (2:1) were collected. Carbon tetrachloride (3 to 4 ml) was added, and the mixture was concentrated under reduced pressure to a volume of about 1 ml. Again, about 4 ml of carbon tetrachloride was added to form a solution having a volume of about 5 ml. About 4 ml of the solution was used for NMR spectroscopy, and about 1 ml of it was used for IR and UV spectroscopy. Each of these portions was concentrated under reduced pressure until the solvent was almost exhausted, and the respective spectra were quickly measured.

UV$\lambda_{max}^{THF}$ nm: 0 (Shoulder), 269.5
IR$\nu_{max}^{CHCl_3}$ $cm^{-1}$: 1778 (C=O of $\beta$-lactam),
1690 (C=O of ester).
NMR ($CDCl_3$) $\delta$:
1.06 (3H, t, J=7.5Hz, —$CH_2$—$CH_3$),
1.59 (1H, s, —SH),
1.55—2.05 (2H, m, —$CH_2$—$CH_3$),
2.70—3.60 (3H, m, C—4$H_2$, C—6H),
3.92 (1H, dt, J=3.0Hz, J=9.0Hz, C—5*H*),
5.22 (1H, d, J=14.0Hz, —*CH*·H—Ar),
5.53 (1H, d, J=14.0Hz, —CH·*H*—Ar),
7.65 (2H, d, J=9.0Hz, ArH),
8.22 (2H, d, J=9.0Hz, ArH).

## Example 25

Production of benzyl 6-ethyl-3-mercapto-7-oxo-1-azabicyclo[3.2.0]hept-2-ene-2-carboxylate:

54.3 mg of PS-5.benzyl ester sulfoxide was dissolved in 15 ml of dry dimethyl formamide. The solution was cooled to −40°C, and with stirring, 1.15 ml of a dimethyl formamide solution of sodium hydrosulfide ($NaSH.XH_2O$, abt 70%) (10 mg/ml) was added gradually with stirring. Five minutes later, the desired product having an Rf of 0.74 (benzene-acetone in a ratio of 1;1) in silica gel TLC was determined. The mixture was stirred at the same temperature for 30 minutes. The reaction mixture was poured into 120 ml of carbon tetrachloride, and washed with a cooled 0.1M phosphate buffer (pH 6.86) (80 ml=3). The carbon tetrachloride solution was dried over anhydrous sodium sulfate, and concentrated under reduced pressure to a volume of about 0.5 ml. About 0.4 ml of the concentrate was used for NMR spectroscopy, and about 0.1 ml of it for IR and UV spectroscopy. The respective spectra were quickly measured.

UV$\lambda_{max}^{THF}$ nm: 314.2
IR$\nu_{max}^{CCl_4}$ $cm^{-1}$: 1783 (C=O of $\beta$-lactam)
1683 (C=O of ester)
NMR ($CCl_4$) $\delta$:
1.03 (3H, t, J=7.5Hz, —$CH_2$—$CH_3$)
1.50—2.05 (2H, m, —$CH_2$—$CH_3$)
2.50—3.50 (3H, m, C—4$H_2$, C—6H)
3.77 (1H, dt, J=3.0Hz, J=9.0Hz, C—5H)
5.12 (1H, d, J=13.0Hz, —*CH*·H—Ar)
5.30 (1H, d, J=13.0Hz, —CH·*H*—Ar)

6.15 (1H, s, —SH)
7.15—7.55 (5H, m, Ar)

Example 26

Production of benzyl 6-isopropyl-3-mercapto-7-oxo-1-azabicyclo[3.2.0]hept-2-ene-2-carboxylate:

Example 25 was repeated except that PS—6.benzyl ester.sulfoxide was used instead of PS—5.benzyl ester.sulfoxide. Thus, the title compound was obtained.

UV$\lambda_{max}^{THF}$ nm: 314
IR$\nu_{max}^{CCl_4}$ cm$^{-1}$: 1782 (beta-lactam), 1684 (ester).

Example 27

Production of pivaloyloxymethyl 6-ethyl-3-mercapto-7-oxo-1-azabicyclo[3.2.0]hept-2-ene-2-carboxylate:

A solution of 47.7 mg of PS—5.pivaloyloxymethyl ester.sulfoxide was dissolved in 15 ml of dry dimethyl formamide. The solution was cooled to −40°C. With stirring, 0.96 ml of a dimethyl formamide solution (10 mg/ml) of sodium hydrosulfide (NaSH.x$H_2O$ abt 70%) was gradually added. The mixture was stirred at the same temperature for 30 minutes. The reaction mixture was poured into 70 ml of benzene, and washed with a cooled 0.1M phosphate buffer (pH 6.86) (40 ml=3). The benzene layer was dried over anhydrous sodium sulfate, concentrated under reduced poressure to a volume of about 0.5 ml, and chromatographed on a column (1.2 x 90 cm) of Biobeads S—X3 using benzene as an eluent. Those eluates which showed an UV absorption at Rf=0.43 in silica TLC with benzene-acetone (1:1) were collected, and 3 to 4 ml of carbon tetrachloride was added. The mixture was concentrated under reduced pressure to a volume of about 1 ml, and again 3 to 4 ml of carbon tetrachloride was added to form a solution having a volume of about 5 ml. About 4 ml of the solution was used for NMR spectroscopy, and about 1 ml of it, for IR and UV spectroscopy. Each of these portions was concentrated under reduced pressure until the solvent was almost exhausted, and the respective spectra were quickly measured.

UV$\lambda_{max}^{THF}$ nm: 316.5
IR$\nu_{max}^{THF}$ cm$^{-1}$: 1779 (C=O of $\beta$-lactam),
1753 (C = O of ester), 1710 (C=O of ester, shoulder).
NMR ($CDCl_2$):
1.03 (3H, t, J=7.5Hz, —$CH_2$—$CH_3$)
1.67 (1H, S, —SH)
1.22 (9H, S, —C($CH_3$)$_3$)
1.57—2.07 (2H, m, —$CH_2$—$CH_3$)
2.50—3.65 (3H, m, C—4$H_2$, C—6H)
3.87 (1H, td, J=3.0Hz, J=9.0Hz, C—5H)
5.85 (1H, d, J=5.0Hz, —O—CH·H—O—)
5.94 (1H, d, J=5.0Hz, —O—CH·H—O—)

Example 28

Production of p-nitrobenzyl (5R, 6S)-6-[(1R)-1-hydroxy]-ethyl-3-mercapto-7-oxo-1-azabicyclo[3.2.0]hept-2-ene-2-carboxylate:

In the same way as in Example 24, there was produced a comopund in which the 3-position was —SH. The compound had an Rf of 0.40 in TLC with benzene-acetone (1:3).

UV$\lambda_{max}^{THF}$: 310.0 (sh), 269.0
IR$\nu_{max}^{CHCl_2}$: 1770 (C=O of $\beta$-lactam),
1680 (C=O of ester)

Example 29

Production of p-nitrobenzyl bis-(6-ethyl-3-thio-7-oxo-1-azabicyclo[3.2.0]hept-2-ene-2-carboxylate:

Sodium hydrosulfide (NaSH.x$H_2O$ abt 70%) was added in an amount of 3.82 ml as a dry dimethyl formamide solution in a concentration of 10 mg/ml to a solution of 204.8 mg of PS—5.p-nitrobenzyl ester.sulfoxide in 70 ml of dry dimethyl formamide at −35°C with stirring. With stirring at the same temperature, 318 microliters of TEA was added 1.5 hours later. Furthermore, 45.7 microliters of trichloroacetonitrile was added. The mixture was stirred at the same temperature for 1 hour to complete the reaction. After the reaction, the reaction mixture was poured into about 300 ml of benzene, and washed with a 0.1M phosphate buffer (pH 8.40) (250 ml x 3). The organic layer was dried over anhydrous sodium sulfate, and the benzene was distilled off under reduced pressure to give a yellowish brown oily product. The oily product was chromatographed on a column (1.0 x 24.0 cm) of silica gel (20 g) using benzene-acetone (15:1) as an eluent. The desired product was searched by TLC, and fractions which showed an UV absorption at Rf=0.33 in silica gel TLC with benzene-acetone (10:1) were collected and distilled under reduced pressure to remove the solvent. Thus, the desired product was obtained in an amount of 86.8 mg (yield 54.9%).

$[\alpha]_D^{23}$: −103.4° (C 0.5 THF)

UV$\lambda_{max}^{THF}$ nm ($\varepsilon$): 270.0 ($\varepsilon$=15400),
325.2 ($\varepsilon$=13800)
IR$\nu_{max}^{CHCl_3}$ cm$^{-1}$: 1780 (C=O of $\beta$-lactam)
1700 (C=O of ester)
NMR ($CDCl_3$) $\delta$:
1.06 (3H, t, J=7.5Hz, —$CH_2CH_3$)
1.60—2.00 (2H, m, —$CH_2CH_3$)
2.98 (1H, dd, J=9Hz, J=19Hz, C—4*H*)
3.42 (1H, dd, J=9Hz, J=19Hz, C—4*H*)
2.90—3.30 (1H, m, C—6*H*)
3.94 (1H, dt, J=3Hz, J=9Hz, C—5*H*)
5.22 (1H, d, J=14Hz, —C*H*·H—Ar)
5.50 (1H, d, J=14Hz, —CH·*H*—Ar)
7.61 (2H, d, J=9Hz, Ar)
8.19 (2H, d, J=9Hz, Ar)
Mass (m/z): 348

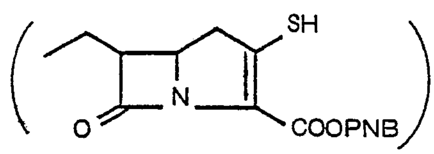

316

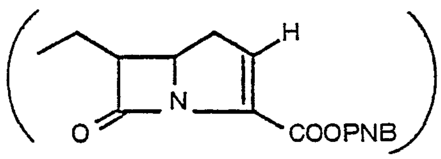

Example 30

Production of p-nitrobenzyl 6-ethyl-3-methylthio-7-oxo-1-azabicyclo[3.2.0]hept-2-ene-2-carboxylate:

(A) 23 mg (0.05 millimole) of PS—5.p-nitrobenzyl ester.sulfoxide was dissolved in 10 ml of anhydrous dimethyl formamide. The solution was cooled to −35°C, and 0.43 ml of a separately prepared DMF solution of sodium hydrosulfide (10 mg/ml) was added gradually. The reaction was performed at the same temperature for 10 minutes. The reaction mixture was poured into 50 ml of ethyl acetate and washed with a phosphate buffer (pH 6.86). The ethyl acetate layer was dried over anhydrous sodium sulfate, and concentrated under reduced pressure to a volume of 10 ml. The concentrate was cooled to 0°C, and 3 ml of an ether solution of diazomethane was added. The mixture was then concentrated to dryness under reduced pressure. The residue was dissolved in a small amount of benzene and adsorbed to a silica gel column (10 ml). The column was eluted with benzene-acetone (30:1). Fractions which showed an UV absorption at Rf=0.78 by silica gel TLC with benzene-acetone (3:1) were collected and concentrated to dryness under reduced pressure to give 10.8 mg of the desired product.

(B) 0.43 ml of a dimethyl formamide solution of sodium hydrosulfide (10 mg/ml) cooled to −35°C was gradually added with stirring to a solution of 23 mg (0.05 millimole) of PS—5.p-nitrobenzyl ester.sulfoxide in 10 ml of anhydrous dimethyl formamide. The reaction was carried out for 10 minutes. Then, at the same temperature, 2.8 microliters (0.20 millimole) of triethylamine and 6.3 microliters (0.10 millimole) of methyl iodide were added. The mixture was stirred further. The reaction mixture was poured into 50 ml of ethyl acetate, and washed with a 0.1M phosphate buffer (pH 6.8). The organic layer was dried over anhydrous sodium sulfate and concentrated under reduced pressure. The resulting residue was isolated and purified in the same manner as described in section (A) above to give 12.1 mg of the desired compound.

$[\alpha]_D^{23}$ +49.8° (C=0.5, THF)
UV$\lambda_{max}^{THF}$ nm ($\varepsilon$): 320 (9600), 269 (9400).
IR$\nu_{max}^{CHCl_3}$ cm$^{-1}$: 1778 (CO of $\beta$-lactam)
1702 (CO of ester).
NMR ($CDCl_3$) $\delta$:
1.04 (3H, t, J=7.0Hz, $CH_2CH_3$)
1.85 (2H, m, $CH_2CH_3$)
2.36 (3H, S, $SCH_3$)
3.12 (3H, m, C—6*H*, C—4$H_2$)
3.94 (1H, dt, J=3.0Hz, J=9.0Hz, C—5*H*)
5.20 (1H, d, J=15.0Hz, C*H*H—Ar)

5.50 (1H, d, J=15.0Hz, CH*H*—Ar)
7.60 (2H, d, J=8.0Hz, Ar) 8.16 (2H, D, J=8.0Hz, Ar)
Mass (m/z): 362 ($M^+$),
292 ($M^+$ —$CH_3CH_2CHCO$).

Example 31

Production of p-nitrobenzyl 3-n-butylthio-6-isopropyl-7-oxo-1-azabicyclo[3.2.0]hept-2-ene-2-carboxylate:

Prepared in the same way as in Example 30 (B).
UV$\lambda_{max}^{THF}$ nm ($\varepsilon$): 322 (11500),
270 (10100).
IR$\nu_{max}^{CHCl_3}$ cm$^{-1}$: 1770 (CO of $\beta$-lactam),
1695 (ester).
NMR ($CDCl_3$) $\delta$: 0.92 (3H, t, J=7.0, —$CH_2CH_2CH_2CH_3$),

1.02 (3H, t, J=7.0, 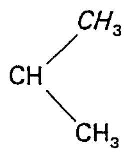 ),

1.05 (3H, t, J=7.0, 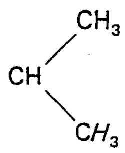 ),

1.20—2.20 (5H, m, 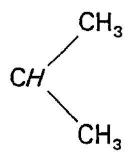 , 2 × $CH_3$),

2.70—3.55 (5H, m, C—4H, C—6, $SCH_2$),
3.95 (1H, dt, J=9.0, J=3.0, C—5H),
5.20 (1H, d, J=14.0, ArC*H*H),
5.50 (1H, d, J=14.0, ArCH*H*),
7.64 (2H, d, J=9.0, Ar*H*),
8.18 (2H, d, J=9.0, Ar*H*).

Example 32

Production of p-nitrobenzyl 3-cyclohexylthio-6-isopropyl-7-oxo-1-azabicyclo[3.2.0]hept-2-ene-2-carboxylate:

Prepared in the same way as in Example 30 (B).
UV$\lambda_{max}^{THF}$ nm ($\varepsilon$): 325 (15000), 270 (12100)
IR$\nu_{max}^{CHCl_3}$ cm$^{-1}$: 1770 (CO of $\beta$-lactam),
1695 (ester)
NMR ($CDCl_3$) $\delta$:

1.02 (3H, d, J=7.0, 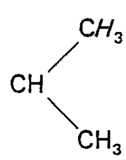 )

1.05 (3H, d, J=7.0, 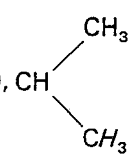 )

1.18—2.30 (11H, m, 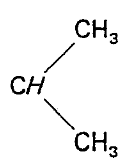 , Cyclohexyl $CH_2$)

2.78—3.30 (4H, m, C—4H, C—6H, SCH)
3.96 (1H, dt, J=9.0, J=3.0, C—4H)
5.20 (1H, d, J=14.0, Ar*CH*H)
5.48 (1H, d, J=14.0, ArCH*H*)
7.63 (2H, d, J=9.0, Ar*H*)
8.20 (2H, d, J=9.0, Ar*H*)

Example 33

Production of p-nitrobenzyl 6-isopropyl-3-(imidazol-4-yl)methylthio-7-oxo-1-azabicyclo[3.2.0]hept-2-ene-2-carboxylate:

22.0 mg of PS—6.p-nitrobenzyl ester.sulfoxide was dissolved in 10 ml of dry dimethyl formamide, and the solution was cooled to −35°C. with stirring, 0.40 ml of a dimethyl formamide solution (10 mg/ml) of sodium hydrosulfide (NaSH.x$H_2O$ abt 70%) was gradually added. The mixture was stirred at the same temperature for 30 minutes to convert the 3-position to —SH. Then, 26.4 microliters of triethylamine was added, and further a solution of 10.9 mg of 4-chloromethylimidazole hydrochloride in 0.5 ml of dimethyl formamide was added. The mixture was stirred at the same temperature for 1 hour. The reaction mixture was poured into 70 ml of benzene, and washed with a 0.1M phosphate buffer (pH 8.40) (40 ml x 3). The washed product was dried over anhydrous sodium sulfate, concentrated under reduced pressure, dried *in vacuo* and chromatographed on a column (1.2 x 85 cm) of Sephadex LH—20 using acetone as a developing solvent. The fractions were analyzed by TLC, and those fractions which contained the desired product having an Rf of 0.15 (benzene-acetone = 1:1 were collected. They were concentrated under reduced pressure, and dried *in vacuo* to give 10.4 mg (yield 51.2%) of the desired product.

UV$\lambda_{max}^{THF}$ nm ($\varepsilon$) 323 (14200) 269 (12000)
IR$\nu_{max}^{CHCl_2}$ $cm^{-1}$: 1777 (C=O of $\beta$-lactam)
1704 (C=O of ester)
NMR ($CDCl_3$) $\delta$:

1.08 (3H, d, J=7.2 Hz, 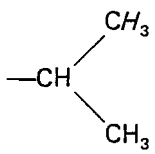

1.12 (3H, d, J=7.2 Hz, 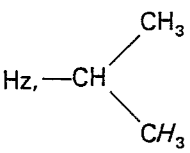

1.5—2.2 (1H, m, 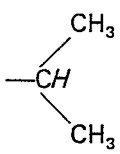 )

2.7—3.5 (3H, m, C—4$H_2$, C—6*H*)
3.95 (1H, dt, J=3Hz, J=9Hz, C—5*H*)
4.01 (2H, S, —S—$CH_2$—)
5.15 (1H, d, J=12.5Hz, —*CH*·H—Ar)
5.40 (1H, d, J=12.5Hz, —CH·*H*—Ar)
5.6—6.1 (1H, broad, >NH)
6.95 (1H, S, Imd-5′ *H*)
7.50 (1H, S, Imd-2′ *H*)
7.55 (2H, d, J=9Hz, ArH)
8.10 (2H, d, J=9Hz, ArH)

Mass (m/z): 442 ($M^+$)

358 ($M^+$—$CH_3$—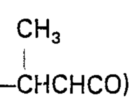)

The following compounds were produced in the same way as describe in Example 30 (B) or Example 33.

Example 34

Production of p-nitrobenzyl 3-(2-ethyloxy)ethylthio-6-isopropyl-7-oxo-1-azabicyclo[3.2.0]hept-2-ene-2-carboxylate:

UV$\lambda_{max}^{THF}$ nm ($\varepsilon$): 320 (13600) 269 (12000)
IR$\nu_{max}^{CHCl_3}$ cm$^{-1}$: 1770 (CO of $\beta$-lactam)
1700 (ester)
NMR ($CDCl_3$) $\delta$:

0.90—1.25 (9H, m, 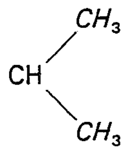 , $CH_2CH_3$)

1.90—2.30 (1H, m, 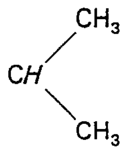 )

2.80—3.75 (9H, m, C—4H, C—6, $CH_2 \times 3$)
3.95 (1H, dt, J=9.0, J=3.0, C—5H)
5.20 (1H, d, J=14.0, ArCH*H*)
5.48 (1H, d, J=14.0, ArCH*H*)
7.62 (2H, d, J=9.0, Ar*H*)
8.18 (2H, d, J=9.0, Ar*H*)

Example 35

Production of p-nitrobenzyl 3-(2-acetoxy)ethylthio-6-isopropyl-7-oxo-1-azabicyclo[3.2.0]hept-2-ene-2-carboxylate:

UV$\lambda_{max}^{THF}$ nm ($\varepsilon$): 317 (9800) 268 (10200)
IR$\nu_{max}^{CHCl_3}$ cm$^{-1}$: 1780 (CO of $\beta$-lactam)
1740 (OAc) 1700 (ester)
NMR ($CDCl_3$) $\delta$:

1.03 (3H, d, J=7.0Hz, 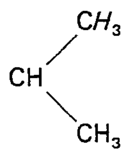 )

1.06 (3H, d, J=7.0, 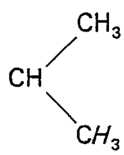 )

1.90—2.30 (1H, m, 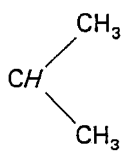 )

2.07 (3H, S, $COCH_3$)
2.70—3.50 (5H, m, $SCH_2$, C—4H, C—6H)
4.00 (1H, dt, J=9.5, J=3.0, C—5H)
4.20 (2H, t, J=7.5, $CH_2OCO$)
5.18 (1H, d, J=14.0, Ar*CH*H)
5.48 (1H, d, J=14.0, ArCH*H*)
7.58 (2H, d, J=9.0, Ar*H*)
8.14 (2H, d, J=9.0, Ar*H*)

Example 36

Production of p-nitrobenzyl 3-(2-dimethylamino)ethylthio-6-isopropyl-7-oxo-1-azabicyclo[3.2.0]hept-2-ene-2-carboxylate:

UV$\lambda_{max}^{THF}$ nm ($\varepsilon$): 322 (12000) 270 (11500)
IR$\nu_{max}^{CHCl_3}$ cm$^{-1}$: 1778 ($\beta$-lactam) 1705 (ester)

NMR ($CDCl_3$) $\delta$:

1.02 (3H, d, J=7.0, 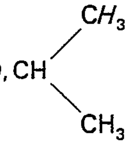 )

1.05 (3H, d, J=7.0, 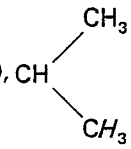 )

1.90—2.20 (1H, m, 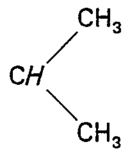 )

2.26 (6H, S, $N(CH_3)_2$)
2.40—3.50 (7H, m, C—4H, C—6H, 2 × $CH_2$)
3.96 (1H, dt, J=9.0, J=3.0, C—5H)
5.22 (1H, d, J=14.0, ArC*H*H)
5.50 (1H, d, J=14.0, ArCH*H*)
7.63 (2H, d, J=9.0, Ar*H*)
8.18 (2H, d, J=9.0, Ar*H*)

Example 37

Production of p-nitrobenzyl 3-(2-phenylacetamido)ethylthio-6-isopropyl-7-oxo-1-azabicyclo[3.2.0]hept-2-ene-2-carboxylate:

$UV\lambda_{max}^{THF}$ nm ($\varepsilon$): 321 (11500), 268 (10000)
$IR\nu_{max}^{CHCl_3}$ $cm^{-1}$: 1775 (CO of $\beta$-lactam) 1700 (ester)
NMR ($CDCl_3$) $\delta$:

1.00 (3H, d, J=7.0, 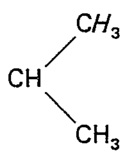 )

1.03 (3H, d, J=7.0, 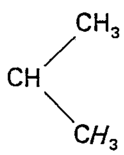 )

1.80—2.25 (1H, m, $CH(CH_3)_2$)
2.70—3.56 (7H, m, C—4H, C—6H, 2 × $CH_2$)
3.60 (2H, S, $COCH_2$)
3.98 (1H, dt, J=9.0, J=3.0, C—5H)
5.23 (1H, d, J=14.0, ArC*H*H)
5.50 (1H, d, J=14.0, ArCH*H*)
5.84 (1H, m, NH)
7.24 (5H, S, Ar*H*)
7.65 (2H, d, J=9.0, Ar*H*)
8.20 (2H, d, J=9.0, Ar*H*)

**Claims**

1. A process for producing a compound of the general formula

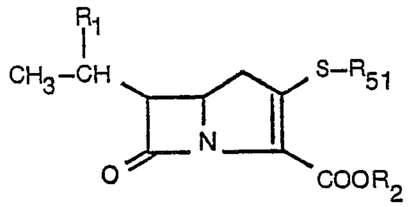

(I—1)

wherein $R_1$ represents a hydrogen atom, a methyl group, or a hydroxyl group, $R_2$ represents a hydrogen atom or an ester residue selected from

(1) a $C_1$—$C_{14}$ alkyl or $C_2$—$C_6$ alkenyl group which may be substituted by a halogen atom, a hydroxyl group, a $C_1$—$C_6$ alkoxy group, a $C_1$—$C_6$ alkylthio group, a $C_3$—$C_{12}$ cycloalkyloxy group, a $C_3$—$C_{13}$ cycloalkylthio group, a phenoxy group (the phenyl group may further be substituted by a halogen atom, a $C_1$—$C_6$ alkoxy group, a $C_1$—$C_6$ haloalkyl group or a nitro group), a phenylthio group (the phenyl group may further be substituted by a halogen atom, a $C_1$—$C_6$ alkoxy group, a $C_1$—$C_6$ haloalkyl group or a nitro group), a $C_2$—$C_{16}$ acyl group, a $C_2$—$C_{14}$ alkoxycarbonyl group (the alkoxy group may further be substituted by a halogen atom), and a $C_8$—$C_{24}$ aralkyloxycarbonyl group (the aralkyl group may further be substituted by a halogen atom, a $C_1$—$C_6$ alkoxy group, a $C_1$—$C_6$ haloalkyl group, or a nitro group);

(2) $C_3$—$C_{15}$ cycloalkyl group;

(3) a phenyl, tolyl, xylyl or naphthyl group which may be substituted by a halogen atom, a $C_1$—$C_6$ alkoxy group, a $C_1$—$C_6$ haloalkyl group, a $C_1$—$C_6$ alkanoylamino group, a $C_1$—$C_6$ alkoxycarbonyl group or a nitro group; and

(4) a $C_1$—$C_6$ alkyl group substituted by 1—3 phenyl groups, and phenyl groups may further be substituted by 1—3 substituents selected from a halogen, $C_1$—$C_6$ alkyl group, $C_1$—$C_6$ alkoxy group, $C_1$—$C_6$ haloalkyl group, $C_1$—$C_6$ alkoxycarbonyl group, hydroxyl group and nitro group,

and $R_{51}$ represents a monovalent organic group selected from groups (1) to (4) as defined for $R_2$, or its salt, which comprises reacting a compound of the general formula

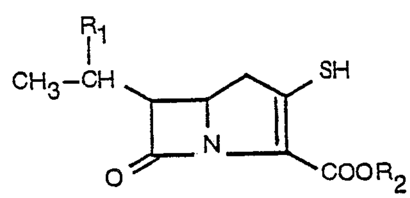

(II)

wherein $R_1$ and $R_2$ are as defined above,
or its salt, with a diazo compound of the formula

$$R_4 = N_2 \qquad \text{(III-2)}$$

wherein $R_4$ represents a divalent group remaining after removal of one hydrogen atom present on the carbon atom at the 1-position from a corresponding $R_{51}$ monovalent organic group in which the carbon atom at the 1-position is a primary or secondary carbon atom.

2. The process of claim 1 wherein the reaction is carried out at a temperature in the range of from −50°C to +50°C.

3. The process of claim 1 wherein the reaction is carried out at room temperature or at lower temperatures.

4. The process of claim 1 wherein the reaction is carried out in an inert organic solvent.

5. The process of claim 1 wherein the compound of formula (III-2) is used in an amount of at least 1 mole per mole of the compound of formula (II) or its salt.

6. The process of claim 1 wherein the compound of formula (III-2) is used in an amount of 1.1 to 2.0 moles per mole of the compound of formula (II) or its salt.

7. The process of claim 1 wherein when $R_2$ in formula (I-1) represents an ester residue easily splittable by hydrolysis or hydrogenolysis, the compound of formula (I-1) is subjected to saponification or hydrogenolysis in a customary manner to form a compound of formula (I-1) wherein $R_2$ is a hydrogen atom or its salt.

8. A process for producing a compound of the general formula

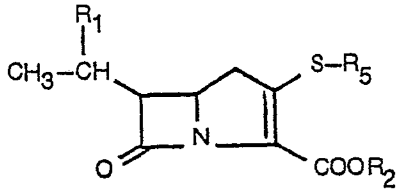

(I)

wherein $R_1$ represents a methyl group, $R_2$ is as defined in claim 1 and $R_5$ represents the group $R_3$ or —CO—$R_3$ in which $R_3$ represents a monovalent organic group selected from groups (1) to (4) as defined for $R_2$,
or its salt, which comprises reacting a compound of the general formula

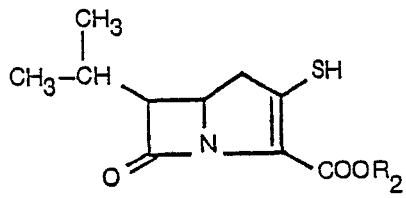

(II-1)

wherein $R_2$ is as defined above,
or its salt, with a compound of the formula

$$R_3—X \qquad (III-1)$$

wherein $R_3$ is as defined above, and X represents a halogen atom or an activated carboxyl group.

9. The process of claim 8 wherein the reaction is carried out at a temperature in the range of from −50°C to +50°C.

10. The process of claim 8 wherein the reaction is carried out at room temperature or at lower temperatures.

11. The process of claim 8 wherein the compound of formula (II-1) or its salt is reacted in the presence of a base with the compound of formula (III-1) wherein X is a halogen atom.

12. The process of claim 8 wherein the reaction is carried out in an inert organic solvent.

13. The process of claim 8 wherein the compound of formula (III-1) is used in an amount of at least 1 mole per mole of the compound of formula (II-1) or its salt.

14. The process of claim 8 wherein the compound of formula (III-1) is used in an amount of 1.1 to 2.0 moles per mole of the compound of formula (II-1) or its salt.

15. The process of claim 8 wherein $R_2$ in formula (I) is an ester residue easily splittable by hydrolysis or hydrogenolysis, the compound of formula (I) is subjected to saponification or hydrogenolysis in a customary manner to form a compound of formula (I) in which $R_2$ is a hydrogen atom, or its salt.

16. A process for producing a compound of the general formula

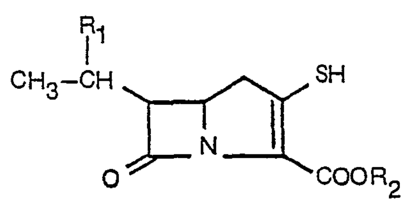

(II)

wherein $R_1$ represents a hydrogen atom, a methyl group, or a hydroxyl group and $R_2$ is as defined in claim 1,
which comprises reacting a compound of the general formula

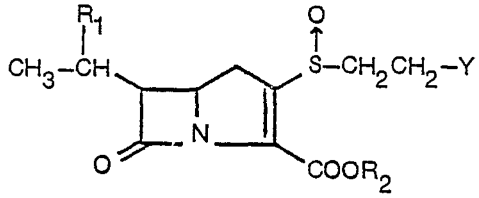

(IV)

wherein Y represents an amino group which may be protected, or its N-oxide, or its salt, with a compound of the formula

$$MSH \quad (V)$$

wherein M represents a hydrogen atom or an alkali metal.

17. The process of claim 16 wherein the reaction is carried out at room temperature or at lower temperatures.

18. The process of claim 17 wherein the reaction is carried out at a temperature in the range of from −30°C to −50°C.

19. The process of claim 16 wherein the compound of formula (iv) or its salt is reacted in the presence of a base with the compound of formula (V) in which M is a hydrogen atom.

20. The process of claim 16 wherein the reaction is carried out in an inert organic solvent.

21. The process of claim 16 wherein the compound of formula (V) is used in an amount of at least 1 mole per mole of the compound of formula (IV) or its salt.

22. The process of claim 16 wherein the compound of formula (V) is used in an amount of 1.1 to 1.5 moles per mole of the compound of formula (IV) or its salt.

23. A compound of the general formula

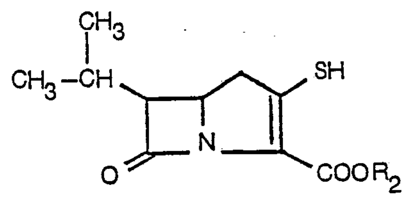

(I-a)

wherein $R_2$ is as defined in claim 1;
and its salt.

**Revendications**

1. Un procédé de production de composés de formule générale ci-dessous

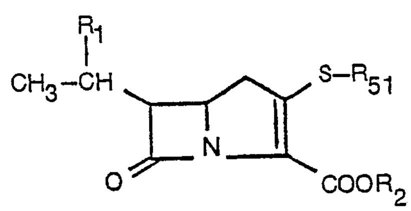

(I-1)

(dans laquelle $R_1$ représente un atome d'hydrogène, un méthyle ou un hydroxyle, $R_2$ un atome d'hydrogène ou un radical choisi parmi

(1) un alkyle en $C_1$—$C_{14}$ ou un alcényle en $C_2$—$C_6$ pouvant être substitué par un atome d'halogène, un hydroxyle, un alcoxy en $C_1$—$C_6$, un alkylthio en $C_1$—$C_6$, un cyclo-alkyloxy en $C_3$—$C_{12}$, un cyclo-alkylthio en $C_3$—$C_{13}$, un phénoxy (le groupe phényle pouvant être lui-même substitué par un atome d'halogène, un alcoxy en $C_1$—$C_6$, un halogéno-alkyle en $C_1$—$C_6$ ou un groupe nitro), un phénylthio (le phényle pouvant être lui-même substitué par un atome d'halogène, un alcoxy en $C_1$—$C_6$, un halogéno-alkyle en $C_1$—$C_6$ ou un groupe nitro), un acyle en $C_2$—$C_{16}$, un alcoxycarbonyle en $C_2$—$C_{14}$ (dont l'alcoxy peut être lui-même substitué par un atome d'halogène), et un aralkyloxycarbonyle en $C_8$—$C_{24}$ (dont l'aralkyle peut être lui-même substitué par un atome d'halogène, un alcoxy en $C_1$—$C_6$, un halogéno-alkyle en $C_1$—$C_6$ ou un groupe nitro);

(2) un cycloalkyle en $C_3$—$C_{15}$;

(3) un phényle, un tolyle, un xylyle ou un naphtyle pouvant être substitués par un atome d'halogène, un alcoxy en $C_1$—$C_6$, un halogéno-alkyle en $C_1$—$C_6$, un alcanoylamino en $C_1$—$C_6$, un alcoxycarbonyle en $C_1$—$C_6$ ou un groupe nitro; et

(4) un alkyle en $C_1$—$C_6$ substitué par 1 à 3 phényles pouvant eux mêmes porter 1 à 3 substituants choisis parmi un halogène, un alkyle en $C_1$—$C_6$, un alcoxy en $C_1$—$C_6$, un halogéno-alkyle en $C_1$—$C_6$, un alcoxycarbonyle en $C_1$—$C_6$, un hydroxyle et un groupe nitro,

et $R_{51}$ représente un groupe organique monovalent choisi parmi les groupes (1) à (4) qui viennent d'être définis pour $R_2$), ou de sels de ces composés, procédé qui comprend la réaction d'un composé de formule générale

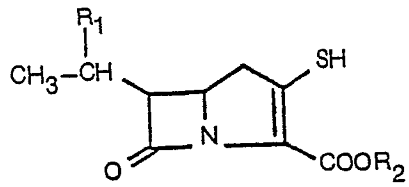

(II)

$R_1$ et $R_2$ ayant les significations ci-dessus, ou d'un sel d'un tel composé, avec un composé diazoïque de formule

$$R_4 = N_2 \qquad \text{(III-2)}$$

$R_4$ représentant un groupe divalent résultant de l'élimination d'un atome d'hydrogène de l'atome de carbone à la position 1 d'un groupe organique monovalent correspondant $R_{51}$ dont l'atome de carbone à la position 1 est un atome de carbone primaire ou secondaire.

2. Procédé selon la revendication 1 dans lequel la réaction est effectuée à une température comprise entre −50°C et +50°C.

3. Procédé selon la revendication 1 dans lequel la réaction est effectuée à la température ordinaire ou à une température plus basse.

4. Procédé selon la revendication 1 dans lequel la réaction est effectuée dans un solvant organique inerte.

5. Procédé selon la revendication 1 dans lequel la proportion employée du composé de formule (III-2) est d'au moins 1 mole par mole du composé de formule (II) ou de son sel.

6. Procédé selon la revendication 1 dans lequel la proportion employée du composé (III-2) est de 1,1 à 2,0 moles par mole du composé (II) ou de son sel.

7. Procédé selon la revendication 1 dans lequel si $R_2$, dans le composé de formule (I-1), est un radical facilement éliminable par hydrolyse ou par hydrogénolyse, on soumet le composé (I-1) à une saponification ou à une hydrogénolyse de la manière courante pour former un composé de formule (I-1) dans lequel $R_2$ est un atome d'hydrogène, ou un sel de ce composé.

8. Un procédé de production de composés de formule générale

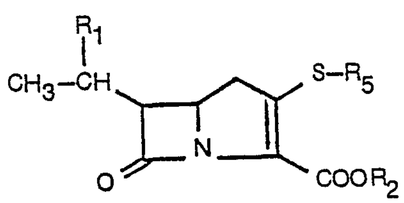

(I)

(dans laquelle $R_1$ représente un groupe méthyle, $R_2$ a la signification donnée à la revendication 1 et $R_5$ représente un groupe $R_3$ ou $—CO—R_3$, $R_3$ étant un groupe organique monovalent choisi parmi les groupes (1) à (4) qui ont été définis pour $R_2$),

ou de sels de ces composés, procédé qui comprend la réaction d'un composé de formule générale

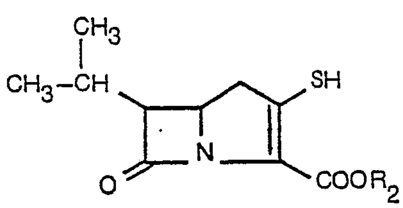

(II-1)

$R_2$ ayant la signification ci-dessus,

ou d'un sel de ce composé, avec un composé de formule

$$R_3—X \qquad \text{(III-1)}$$

$R_3$ ayant la signification ci-dessus et X représentant un atome d'halogène ou un groupe carboxylique activé.

9. Procédé selon la revendication 8 dans lequel la réaction est effectuée entre −50°C et +50°C.

10. Procédé selon la revendication 8 dans lequel la réaction est effectuée à la température ordinaire ou à une température plus basse.

11. Procédé selon la revendication 8 dans lequel on fait réagir le composé de formule (II-1) ou son sel, en présence d'une base, avec un composé de formule (III-1) dans lequel X est un atome d'halogène.

12. Procédé selon la revendication 8 dans lequel la réaction est effectuée dans un solvant organique inerte.

13. Procédé selon la revendication 8 dans lequel le composé de formule (III-1) est employé dans une proportion d'au moins 1 mole par mole du composé de formule (II-1) ou de son sel.

14. Procédé selon la revendication 8 dans lequel le composé de formule (III-1) est employé dans une proportion de 1,1 à 2,0 moles par mole du composé de formule (II-1) ou de son sel.

15. Procédé selon la revendication 8 dans lequel si $R_2$, dans le composé de formule (I), est un radical facilement éliminable par hydrolyse ou hydrogénolyse, ou soumet ce composé (I) à une saponification ou à une hydrogénolyse de la manière courante pour former un composé de formule (I) dans lequel $R_2$ est un atome d'hydrogène, ou un sel de ce composé.

16. Un procédé de production de composés de formule générale

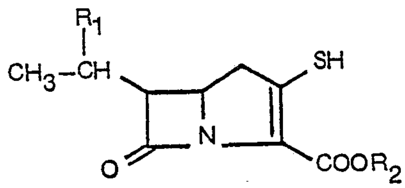

(II)

(dans laquelle $R_1$ représente un atome d'hydrogène, un méthyle ou un hydroxyle et $R_2$ a la signification donnée à la revendication 1),

procédé qui comprend la réaction d'un composé de formule générale

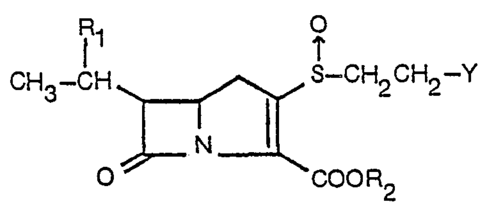

(IV)

Y représentant un groupe amino pouvant être protégé ou son oxyde sur l'azote, ou bien d'un sel de ce composé,

avec un composé de formule

MSH (V)

M représentant un atome d'hydrogène ou un métal alcalin.

17. Procédé selon la revendication 16 dans lequel la réaction est effectuée à la température ordinaire ou à une température plus basse.

18. Procédé selon la revendication 17 dans lequel la réaction est effectuée entre −30°C et −50°C.

19. Procédé selon la revendication 16 dans lequel on fait réagir le composé de formule (IV) ou son sel, en présence d'une base, avec un composé de formule (V) dans lequel M est un atome d'hydrogène.

20. Procédé selon la revendication 16 dans lequel la réaction est effectuée dans un solvant organique inerte.

21. Procédé selon la revendiction 16 dans lequel le composé de formule (V) est employé dans une proportion d'au moins 1 mole par mole du composé (IV) ou de son sel.

22. Procédé selon la revendication 16 dans lequel le composé (V) est employé dans une proportion de 1,1 à 1,5 mole par mole du composé (IV) ou de son sel.

23. Les composés de formule générale

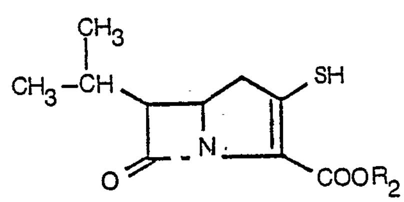

(I-a)

dans lesquels $R_2$ a la signification donnée à la revendication 1, ainsi que les sels de ces composés.

**Patentansprüche**

1. Verfahren zur Herstellung einer Verbindung der allgemeinen Formel:

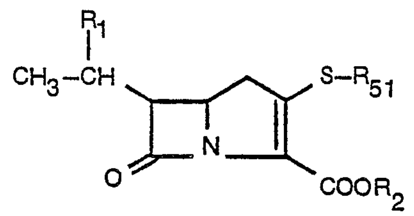

(I-1)

worin $R_1$ für ein Wasserstoffatom, eine Methylgruppe oder eine Hydroxylgruppe steht, $R_2$ für ein Wasserstoffatom oder einen Esterrest, ausgewählt aus

(1) einer $C_1$—$C_{14}$-Alkyl- oder $C_2$—$C_6$-Alkenylgruppe, die durch ein Halogenatom, eine Hydroxylgruppe, eine $C_1$—$C_6$-Alkoxygruppe, eine $C_1$—$C_6$-Alkylthiogruppe, eine $C_3$—$C_{12}$-Cycloalkyloxygruppe, eine $c_3$—$C_{13}$-Cycloalkylthiogruppe, eine Phenoxygruppe (wobei die Phenylgruppe weiterhin durch ein Halogenatom, eine $C_1$—$C_6$-Alkoxygruppe, eine $C_1$—$C_6$-Halogenalkylgruppe oder eine Nitrogruppe substituiert sein kann), eine Phenylthiogruppe (wobei die Phenylgruppe weiterhin durch ein Halogenatom, eine $C_1$—$C_6$-Alkoxygruppe, eine $C_1$—$C_6$-Halogenalkylgruppe oder eine Nitrogruppe substituiert sein kann), eine $C_2$—$C_{16}$-Acylgruppe, eine $C_2$—$C_{14}$-Alkoxycarbonylgruppe (wobei die Alkoxygruppe weiterhin durch ein Halogenatom substituiert sein kann) und eine $C_8$—$C_{24}$-Aralkyloxycarbonylgruppe (wobei die Aralkylgruppe weiterhin durch ein Halogenatom, eine $C_1$—$C_6$-Alkoxygruppe, eine $C_1$—$C_6$-Halogenalkylgruppe oder eine Nitrogruppe substituiert sein kann) substituiert sein kann,

(2) einer $C_3$—$C_{15}$-Cycloalkylgruppe,

(3) einer Phenyl-, Tolyl-, Xylyl- oder Naphthylgruppe, die durch ein Halogenatom, eine $C_1$—$C_6$-Alkoxygruppe, eine $C_1$—$C_6$-Halogenalkylgruppe, eine $C_1$—$C_6$-Alkanoylaminogruppe, eine $C_1$—$C_6$-Alkoxycarbonylgruppe oder eine Nitrogruppe substituiert sein kann, und

(4) einer $C_1$—$C_6$-Alkylgruppe, die durch 1 bis 3 Phenylgruppen substituiert ist, wobei die Phenylgruppen weiterhin durch 1 bis 3 Substituenten, ausgewählt aus einem Halogen, einer $C_1$—$C_6$-Alkylgruppe, einer $C_1$—$C_6$-Alkoxygruppe, einer $C_1$—$C_6$-Halogenalkylgruppe, einer $C_1$—$C_6$-Alkoxycarbonylgruppe, einer Hydroxylgruppe und einer Nitrogruppe, substituiert sein können,

steht und $R_{51}$ für eine einwertige organische Gruppe, ausgewählt aus den Gruppen (1) bis (4), wie für $R_2$ definiert, steht, oder ihres Salzes, dadurch gekennzeichnet, daß man eine Verbindung der allgemeinen Formel:

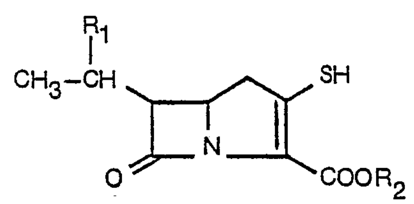

(II)

worin $R_1$ und $R_2$ wie oben definiert sind, oder ihr Salz mit einer Diazoverbindung der Formel

$$R_4{=}N_2 \qquad \text{(III-2)}$$

worin $R_4$ für eine zweiwertige Gruppe steht, die nach Entfernung eines auf dem Kohlenstoffatom in 1-Stellung vorhandenen Wasserstoffatoms von einer entsprechenden einwertigen organischen $R_{51}$-Gruppe, bei der das Kohlenstoffatom in 1-Stellung ein primäres oder sekundäres Kohlenstoffatom ist, zurückbleibt, umsetzt.

2. Verfahren nach Anspruch 1, dadurch gekennzeichnet, daß man die Reaktion bei einer Temperatur im Bereich von −50 bis +50°C durchführt.

3. Verfahren nach Anspruch 1, dadurch gekennzeichnet, daß man die Reaktion bei Raumtemperatur oder bei niedrigeren Temperaturen durchführt.

4. Verfahren nach Anspruch 1, dadurch gekennzeichnet, daß man die Reaktion in einem inerten organischen Lösungsmittel durchführt.

5. Verfahren nach Anspruch 1, dadurch gekennzeichnet, daß man die Verbindung der Formel (III-2) in einer Menge von mindestens 1 mol pro mol der Verbindung der Formel (II) oder ihres Salzes einsetzt.

6. Verfahren nach Anspruch 1, dadurch gekennzeichnet, daß man die Verbindung der Formel (III-2) in einer Menge von 1,1 bis 2,0 mol pro mol der Verbindung der Formel (II) oder ihres Salzes einsetzt.

7. Verfahren nach Anspruch 1, dadurch gekennzeichnet, daß man, wenn $R_2$ in der Formel (I-1) für einen durch Hydrolyse oder Hydrogenolyse leicht abspaltbaren Esterrest steht, die Verbindung der

Formel (I-1) einer Verseifung oder Hydrogenolyse in üblicher Weise unterwirft, um eine Verbindung der Formel (I-1), bei der $R_2$ ein Wasserstoffatom ist, oder ihr Salz zu bilden.

8. Verfahren zur Herstellung einer Verbindung der allgemeinen Formel:

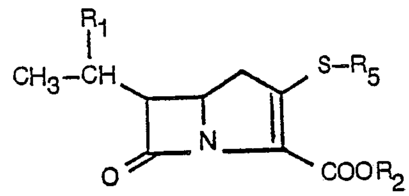

(I)

worin $R_1$ für eine Methylgruppe steht, $R_2$ wie im Anspruch 1 definiert ist und $R_5$ für die Gruppe $R_3$ oder —CO—$R_3$ steht, wobei $R_3$ eine einwertige organische Gruppe, ausgewählt aus den Gruppen (1) bis (4) wie für $R_2$ definiert, ist, oder ihres Salzes, dadurch gekennzeichnet, daß man eine Verbindung der allgemeinen Formel:

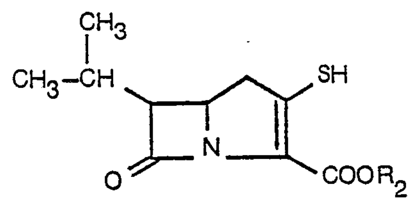

(II-1)

worin $R_2$ wie oben definiert ist, oder ihr Salz mit einer Verbindung der Formel:

$$R_3—X \qquad \text{(III-1)}$$

worin $R_3$ wie oben definiert ist, und X für ein Halogenatom oder eine aktivierte Carboxylgruppe steht, umsetzt.

9. Verfahren nach Anspruch 8, dadurch gekennzeichnet, daß man die Reaktion bei einer Temperatur im Bereich von −50 bis +50°C durchführt.

10. Verfahren nach Anspruch 8, dadurch gekennzeichnet, daß man die Reaktion bei Raumtemperatur oder niedrigeren Temperaturen durchführt.

11. Verfahren nach Anspruch 8, dadurch gekennzeichnet, daß man die Verbindung der Formel (II-1) oder ihr Salz in Gegenwart einer Base mit einer Verbindung der Formel (III-1), wobei X für ein Halogenatom steht, umsetzt.

12. Verfahren nach Anspruch 8, dadurch gekennzeichnet, daß man die Reaktion in einem inerten organischen Lösungsmittel durchführt.

13. Verfahren nach Anspruch 8, dadurch gekennzeichnet, daß man die Verbindung der Formel (III-1) in einer Menge von mindestens 1 mol pro mol der Verbindung der Formel (II-1) oder ihres Salzes einsetzt.

14. Verfahren nach Anspruch 8, dadurch gekennzeichnet, daß man die Verbindung der Formel (III-1) in einer Menge von 1,1 bis 2,0 mol pro mol der Verbindung der Formel (II-1) oder ihres Salzes einsetzt.

15. Verfahren nach Anspruch 8, dadurch gekennzeichnet, daß man, wenn $R_2$ in der Formel (I) für einen leicht durch Hydrolyse oder Hydrogenolyse abspaltbaren Esterrest steht, die Verbindung der Formel (I) in üblicher Weise einer Verseifung oder Hydrogenolyse unterwirft, um eine Verbindung der Formel (I), bei der $R_2$ für ein Wasserstoffatom steht, oder ihr Salz zu erhalten.

16. Verfahren zur Herstellung einer Verbindung der allgemeinen Formel:

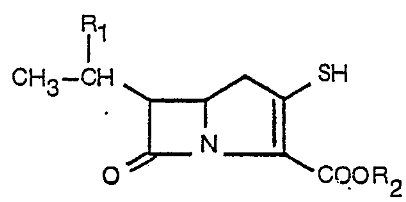

(II)

worin $R_1$ für ein Wasserstoffatom, eine Methylgruppe oder eine Hydroxylgruppe steht und $R_2$ wie im Anspruch 1 definiert ist, dadurch gekennzeichnet, daß man eine Verbindung der allgemeinen Formel:

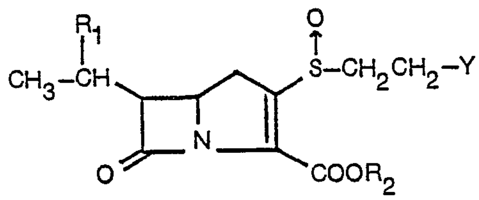

(IV)

worin Y für eine Aminogruppe, die geschützt sein kann, oder ihr N-Oxid steht, oder ihr Salz mit einer Verbindung der Formel:

$$MSH \quad (V)$$

worin M für ein Wasserstoffatom oder ein Alkalimetall steht, umsetzt.

17. Verfahren nach Anspruch 16, dadurch gekennzeichnet, daß man die Reaktion bei Raumtemperatur oder niedrigeren Temperaturen durchführt.

18. Verfahren nach Anspruch 17, dadurch gekennzeichnet, daß man die Reaktion bei einer Temperatur im Bereich von —30 bis —50°C durchführt.

19. Verfahren nach Anspruch 16, dadurch gekennzeichnet, daß man die Verbindung der Formel (IV) oder ihr Salz in Gegenwart einer Base mit einer Verbindung der Formel (V), worin M für ein Wasserstoffatom steht, umsetzt.

20. Verfahren nach Anspruch 16, dadurch gekennzeichnet, daß man die Reaktion in einem inerten organischen Lösungsmittel durchführt.

21. Verfahren nach Anspruch 16, dadurch gekennzeichnet, daß man die Verbindung der Formel (V) in einer Menge von mindestens 1 mol pro mol der Verbindung der Formel (IV) oder ihres Salzes einsetzt.

22. Verfahren nach Anspruch 16, dadurch gekennzeichnet, daß man die Verbindung der Formel (V) in einer Menge von 1,1 bis 1,5 mol pro mol der Verbindung der Formel (IV) oder ihres Salzes einsetzt.

23. Eine Verbindung der allgemeinen Formel:

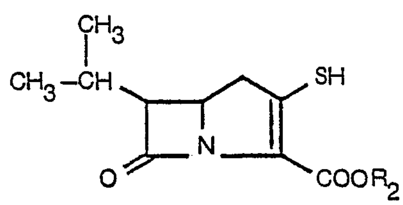

(I-a)

worin $R_2$ wie im Anspruch 1 definiert ist, und ihr Salz.